# EUROPEAN PATENT SPECIFICATION

(11) **EP 3 710 599 B1**
(45) Date of publication and mention of the grant of the patent: **17.07.2024**
(21) Application number: 18808196.2
(22) Date of filing: 09.11.2018
(51) Int. Cl.: C12Q 1/6886, A61P 1/16, G01N 33/50, C12Q 1/6883

(54) **METHOD FOR STAGING LIVER FIBROSIS IN NASH PATIENTS**
VERFAHREN ZUR EINSTUFUNG DER LEBERFIBROSE BEI NASH-PATIENTEN
PROCÉDÉ DE STADIFICATION DE LA FIBROSE HÉPATIQUE CHEZ LES PATIENTS ATTEINTS DE NASH

(30) Priority: 13.11.2017 US 201762585421 P; 01.10.2018 US 201862739778 P
(43) Date of publication of application: 23.09.2020
(73) Proprietor: Gilead Sciences, Inc., Foster City, CA 94404 (US)
(72) Inventor: BILLIN, Andrew, Nicholas, Foster City California 94404 (US); CHUANG, Jen-Chieh, Foster City California 94404 (US); XU, Ren, Y., San Mateo California 94403 (US)
(74) Representative: Marks & Clerk LLP
(86) International application number: PCT/US2018/060106
(87) International publication number: WO 2019/094777

(56) References cited:
- WO-A1-2012/105590
- WO-A1-2014/057522
- WO-A1-2017/082943
- WO-A1-2017/139254
- WO-A1-2017/167934
- WO-A2-2005/039397
- WO-A2-2007/130636
- WO-A2-2011/140093

## Description

### BACKGROUND

NASH represents a significant and growing unmet medical need with no currently approved therapies. An estimated 16 million adults in the United States have NASH. Approximately 25% of patients diagnosed with NASH have advanced liver fibrosis, which is associated with increased morbidity and mortality.

Biopsy is considered a standard for assessments of liver health including NASH. However, biopsy is an invasive technique that is not ideal for many patients. Non-invasive methods may provide advantages for diagnosing, monitoring, or predicting NASH. Described herein are non-invasive markers associated with NASH or a related presentation.

WO 2012/105590 A1 discloses a determination marker comprising YKL-40 which can be used for distinguishing between simple steatosis and NASH in a non-alcoholic fatty liver disease (NAFLD) patient. WO 2005/039397 A2 discloses a method of diagnosing the presence or severity of tissue fibrosis in an individual by detecting α2-macroglobulin (α2-MG) in a sample from the individual, detecting hyaluronic acid (HA) in a sample from the individual, detecting tissue inhibitor of metalloproteinase-1 (TIMP-1) in a sample from the individual, and diagnosing the presence or severity of tissue fibrosis in the individual based on the presence or level of α2-MG, HA and TIMP-1. WO 2017/139254 A1 discloses methods, compositions and kits for determining whether a subject has NAFLD, as well as methods, compositions and kits for determining whether a subject has NASH. WO 2017/167934 A1 discloses a method for the diagnosis of NASH, and for classifying a subject as a potential receiver of a treatment for NASH.

### SUMMARY

The invention is defined by the appended claims. The present disclosure is based on the evaluation of protein and bile acid markers whose levels correlate with the stage, status or treatment outcome of liver diseases or conditions. In one embodiment, provided is a method for classifying a non-alcoholic steatohepatitis (NASH) patient with severe fibrosis, comprising measuring the protein levels of Collectin Kidney 1 (CL-K1), Spondin-1(RSPO1), Matrix metallopeptidase 7 (MMP-7), Complement component 7 (C7), Insulin-like growth factor binding protein 7 (IGFBP7), Interleukin 5 receptor subunit alpha (IL-5Ra), and Thrombospondin-2 (TSP2) in a serum sample isolated from the NASH patient; determining whether Nonalcoholic Steatohepatitis Clinical Research Network (CRN) fibrosis stage of liver fibrosis in the NASH patient is stage 3 or 4, based on the measured protein levels and predetermined reference values, wherein the predetermined reference values include reference protein levels for each of Collectin Kidney 1 (CL-K1), Spondin-1(RSPO1), Matrix metallopeptidase 7 (MMP-7), Complement component 7 (C7), Insulin-like growth factor binding protein 7 (IGFBP7), Interleukin 5 receptor subunit alpha (IL-5Ra), and Thrombospondin-2 (TSP2) at CRN fibrosis stages 0 to 4; and classifying the NASH patient with severe fibrosis if the determined CRN fibrosis stage is 3 or 4.

### BRIEF DESCRIPTION OF THE DRAWINGS

**FIG. 1** shows proteins whose expression levels in the serum significantly correlated with NASH disease severity.
**FIG. 2** is a Venn diagram showing the overlaps between fibrosis stage and NAS components for the protein markers.
**FIG. 3** shows that the diagnostic ability of fibrosis stage at baseline using combined biomarkers is increased compared to the individual best markers.

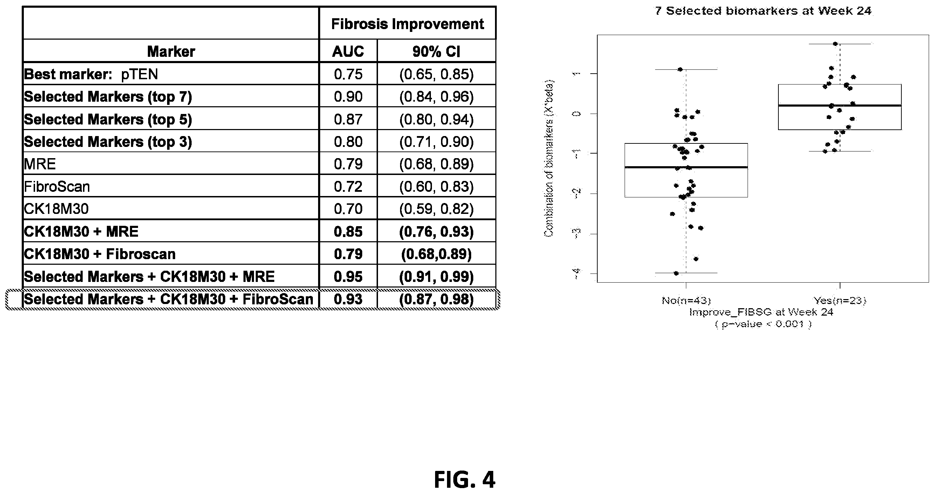

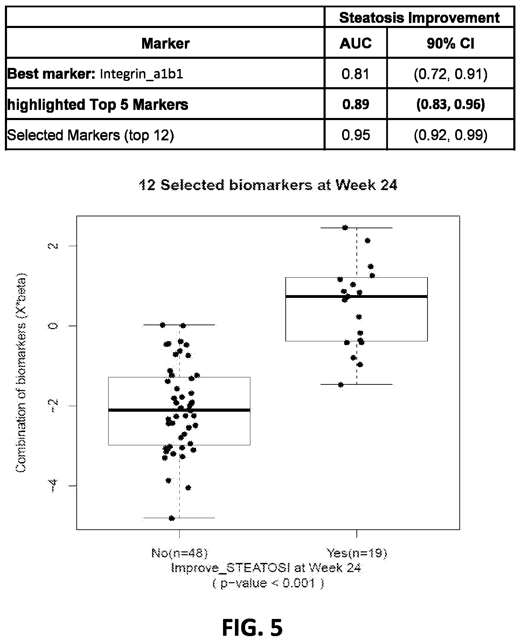

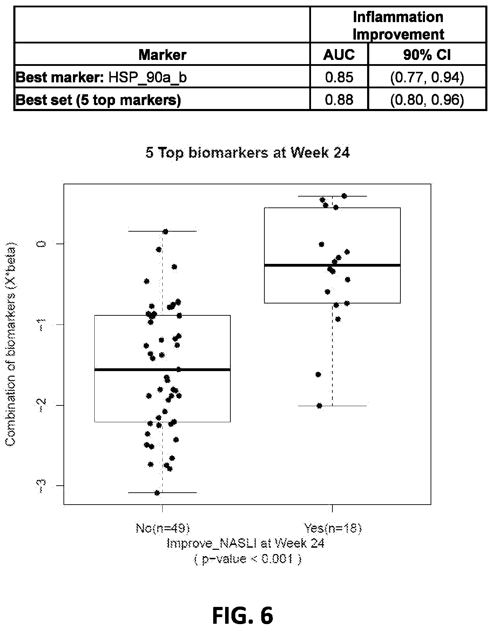

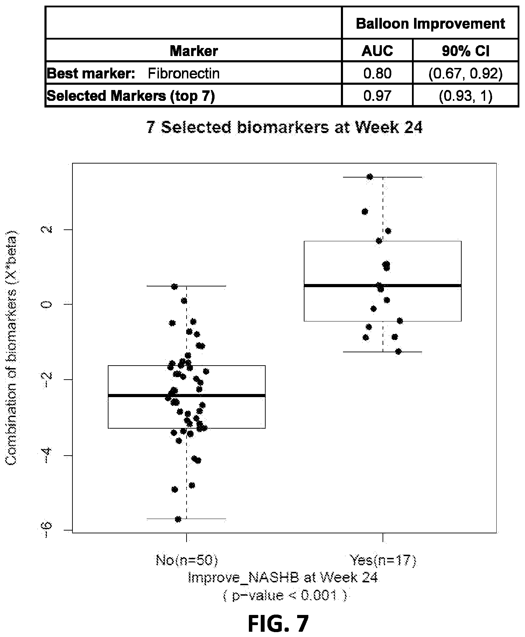

**FIG. 4-7** show the performance of multivariate protein markers for monitoring improvement of clinical parameters, CRN fibrosis stage (FIG. 4), steatosis (FIG. 5), lobular inflammation (FIG. 6), and hepatic ballooning (FIG. 7).
**FIG. 8** (not according to the invention) presents a chart showing that serum bile acid profiles are significantly altered in NASH subjects with different fibrosis stage.
**FIG. 9** (not according to the invention) shows that Serum C4 (7α-hydroxy-4-cholesten-3-one) levels are increased in F2/F3 NASH subjects but are decreased in F4 and decompensated subjects. **p<0.01, ***p<0.001, ****p<0.0001.
**FIG. 10** shows unique and overlapping potential secreted/leaked from multiple databases.
**FIG. 11****.** NASH secretome workflow.
**FIG. 12****.** Diagnosis of severe fibrosis or cirrhosis using hepatic RNA levels of top combined and individual secretome candidates.
**FIG. 13****.** Secretome candidates demonstrated significant association between circulating protein levels and liver fibrosis in 1497 and sim study samples (F2-4).
**FIG. 14-15** (not according to the invention) show the significant correlation between circulating proteins GDF-15 and certain NASH stages and characteristics.
**FIG. 16-18** (not according to the invention) show the significant correlation between circulating proteins CD 163 and certain NASH stages and characteristics.
**FIG. 19** is a Venn diagram showing overlapped metabolite markers for different NASH phenotypes.

It will be recognized that some or all of the figures are schematic representations for purpose of illustration.

### DETAILED DESCRIPTION

### Definitions

Unless defined otherwise, all technical and scientific terms used herein have the same meaning as is commonly understood by one of ordinary skill in the art to which this invention belongs. The headings provided herein are for convenience only and not as limitation in any way.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

Reference throughout this specification to "some embodiments," "one embodiment," "an embodiment," "another embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

As used herein, the term "sample" refers generally to a fluid from a human. Non-limiting examples of a sample include: bile, blood, blood plasma, serum, breast milk, feces, pus, saliva, sebum, semen, sweat, tears, urine, and vomit. In some embodiments, the sample is serum.

As used herein, the term "subject" refers to a mammalian subject. Exemplary subjects include, but are not limited to humans, monkeys, dogs, cats, mice, rats, cows, horses, goats and sheep. In some embodiments, the subject has a liver disease or condition and can be treated as described herein.

As used herein, the term "suspected" when referencing a patient refers to the potential for a patient to have a certain liver disease or condition based on a correlate.

As used herein, the term "treatment," "treating," or similar language refers to a process to (1) delay onset of a disease that is causing clinical symptoms; (2) inhibiting a disease, that is, arresting the development of clinical symptoms; and/or (3) relieving the disease, that is, causing the regression of clinical symptoms or the severity thereof.

As used herein, the term "liver disease or condition" refers to any one or more of the following: liver fibrosis, alcoholic hepatitis, C7 steatohepatitis (NASH), non-alcoholic fatty liver disease (NAFLD), or liver inflammation.

As used herein, the term "NAS" refers to the NAFLD Activity Score, which is a scoring system for NAFLD.

### Identification and Treatment of Liver Diseases or Conditions

The experimental examples of the present disclosure identified non-invasive markers from serum samples that can be used to diagnose liver diseases or conditions, such as liver fibrosis, non-alcoholic steatohepatitis (NASH), non-alcoholic fatty liver disease (NAFLD), and liver inflammation. More specifically, the instant inventors demonstrated that the expression levels of certain protein markers, individually or in combination, significantly correlated with the stage or status of the liver disease or condition. In addition, the serum C4 (7α-hydroxy-4-cholesten-3-one) levels, which were measured to reflect hepatic bile acid biosynthesis, correlated with fibrosis stages and development of cirrhosis.

It is to be understood that information obtained using the diagnostic assays described herein may be used alone or in combination with other information, such as, but not limited to, genotypes or expression levels of other proteins, clinical chemical parameters, histopathological parameters, or age, gender and weight of the subject. When used alone, the information obtained using the diagnostic assays described herein is useful in determining or identifying the clinical outcome of a treatment, selecting a patient for a treatment, or treating a patient, etc. When used in combination with other information, on the other hand, the information obtained using the diagnostic assays described herein is useful in aiding in the determination or identification of clinical outcome of a treatment, aiding in the selection of a patient for a treatment, or aiding in the treatment of a patient and etc. In a particular aspect, the genotypes or expression levels of one or more proteins as disclosed herein are used in a panel of proteins, each of which contributes to the final diagnosis, prognosis or treatment.

Protein markers have also been identified that correlate with clinical improvements following a treatment. These markers, therefore, can be used to monitor the treatment of patients. For example, when the markers show that a treatment has been effective in a patient, the patient may be instructed to continue the treatment. By contrast, if the markers show that no desired improvements have been achieved with the treatment, then a new treatment (e.g., a new medicine or a higher dose) may be used.

In accordance with the invention, therefore, provided is a method of determining the stage or status of liver disease or condition in a human subject, e.g., one that is suspected to have a liver disease or condition. The method entails measuring the expression levels of proteins/genes, selected from Table 1A, in a biological sample isolated from the human subject and determining the stage of liver fibrosis in the human subject based on the expression levels. Specifically, the invention provides a method for classifying a non-alcoholic steatohepatitis (NASH) patient with severe fibrosis, comprising measuring the protein levels of Collectin Kidney 1 (CL-K1), Spondin-1(RSPO1), Matrix metallopeptidase 7 (MMP-7), Complement component 7 (C7), Insulin-like growth factor binding protein 7 (IGFBP7), Interleukin 5 receptor subunit alpha (IL-5Ra), and Thrombospondin-2 (TSP2) in a serum sample isolated from the NASH patient; determining whether Nonalcoholic Steatohepatitis Clinical Research Network (CRN) fibrosis stage of liver fibrosis in the NASH patient is stage 3 or 4, based on the measured protein levels and predetermined reference values, wherein the predetermined reference values include reference protein levels for each of Collectin Kidney 1 (CL-K1), Spondin-1(RSPO1), Matrix metallopeptidase 7 (MMP-7), Complement component 7 (C7), Insulin-like growth factor binding protein 7 (IGFBP7), Interleukin 5 receptor subunit alpha (IL-5Ra), and Thrombospondin-2 (TSP2) at CRN fibrosis stages 0 to 4; and classifying the NASH patient with severe fibrosis if the determined CRN fibrosis stage is 3 or 4. The determination comprises comparing the measured protein levels to reference levels. In some embodiments, the reference levels are obtained from a human subject not suffering from liver fibrosis.

For instance, Table 1A shows that the protein marker "Collectin Kidney 1" has expression levels 5987.2, 4903.3, 8174.95, 11267.55, and 15604.5 (unit: relative fluorescent unit (RFU)), at CRN fibrosis stages 0-4, respectively in the test sample. When a new subject is tested and the expression level of Collectin Kidney 1 is 11000 (RFU, normalized), a determination can be made that this new subject likely has a liver fibrosis at CRN fibrosis stage 3.

In the invention, the proteins are selected from Table 1A or Table 2A. The diagnostic variable, in this aspect, is a CRN fibrosis stage. In other disclosures which do not form part of the present invention, the proteins are selected from Table 1B or 2B, and the diagnostic variable is an Ishak Fibrosis stage; the proteins are selected from Table 1C or 2C, and the diagnostic variable is a NAS score; the proteins are selected from Table 1D or 2D, and the diagnostic variable is a steatosis; the proteins are selected from Table 1E or 2E, and the diagnostic variable is lobular inflammation (LI); or the proteins are selected from Table 1F or 2F, and the diagnostic variable is hepatic ballooning (HB).

It can be seen from the tables that some protein markers are associated with multiple diagnostic variables while some are unique to a particular variable (see summary in Table A). For instance, 6Ckine, BSSP4, IL-8, LIF sR, LTBP4, MIC-1, SAP, SEM6B, SLAF7, and Spondin-1 are unique to CRN fibrosis and HSP 70 is common to all five parameters in Table 5. Accordingly, a suitable expression level of 6Ckine may indicate a particular CRN fibrosis stage, while a suitable expression level of HSP 70 implicates a particular stage or status for all five variables.

As shown in the multivariant analysis of Example 1, a group of seven protein markers, when used in combination, had even greater diagnostic capability. These seven protein markers include C7 (Complement component 7), CL-K1 (Collectin Kidney 1), IGFBP7 (Insulin-like growth factor binding protein 7), Spondin 1 (RSPO1), IL-5Ra (UniProt: Q01344; Interleukin 5 receptor subunit alpha), MMP-7 (Matrix metallopeptidase 7) and TSP2 (Thrombospondin-2). In the invention, all seven of the seven markers are used.

As Example 2 (not according to the invention) has demonstrated, the serum levels of C4 (7α-hydroxy-4-cholesten-3-one) correlate with the stage and status of liver diseases as well, which can individually, or in combination with other markers such as those disclosed herein, be used for the purpose of making diagnosis for liver diseases or conditions.

In addition or independently, the disease assessment can be made with information obtained through conventional or non-conventional methods. In one disclosure, a method (not according to the invention) is provided for identifying a non-alcoholic steatohepatitis (NASH) patient as likely suffering advanced fibrosis. The method entails measuring the AST level, ALT level and platelet count for the NASH patient and calculating a Fibrosis-4 (FIB-4) index, measuring the serum concentrations of tissue inhibitor of metalloproteinases 1 (TIMP-1), amino-terminal propeptide of type III procollagen (PIIINP) and hyaluronic acid (HA) for the NASH patient and calculating an enhanced liver fibrosis (ELF) score, or conducting a transient elastographic (Fibroscan) of the NASH patient to determine a Fibroscan score, and comparing the FIB-4 index and either or both of the ELF score and the Fibroscan score to reference values, and identifying the NASH patient as likely suffering from advanced fibrosis based on the comparison.

In some dislosures, the FIB-4 index and the ELF score are determined. In some disclosures, the FIB-4 index, the ELF score and the Fibroscan score are determined. In some disclosures, no invasive measurements are made to the NASH patient. In one disclosure, an assessment of the FIB-4 or ELF score is made and a patient is treated with one or more therapeutic agents selected from an ASK1 inhibitor, an ACC inhibitor, or an FXR agonist. In one disclosure, an assessment of the FIB-4 or ELF score is made and a patient is treated with selonsertib.

As demonstrated in Example 6 (not according to the invention), metabolites measured in serum samples of an individual can also be used as biomarkers for assessing disease stages. The present disclosure, therefore, discloses a method of determining the stage or status of liver disease or condition in a human subject, e.g., one that is suspected to have a liver disease or condition. The method entails measuring the levels of one or more metabolites, selected from Tables 15-27, in a biological sample (e.g., serum) obtained from the human subject and determining the stage of liver fibrosis in the human subject based on the levels. In some disclosures, the determination comprises comparing the levels to reference levels. In some disclosures, the reference levels are obtained from a human subject not suffering from liver fibrosis.

In some disclosures, the metabolites are selected from Table 15. The diagnostic variable, in this aspect, is a CRN fibrosis stage. In other disclosures which do not form part of the present invention, the metabolites are selected from Table 16, and the diagnostic variable is a NAS score; the metabolites are selected from Table 17, and the diagnostic variable is a steatosis; the metabolites are selected from Table 19, and the diagnostic variable is lobular inflammation (LI); or the metabolites are selected from Table 18, and the diagnostic variable is hepatic ballooning (HB).

In some embodiments, determination of a liver disease or condition in a patient is followed by treatment as described herein. In other embodiments, determination of a liver disease or condition is conducted during the course of treatment.

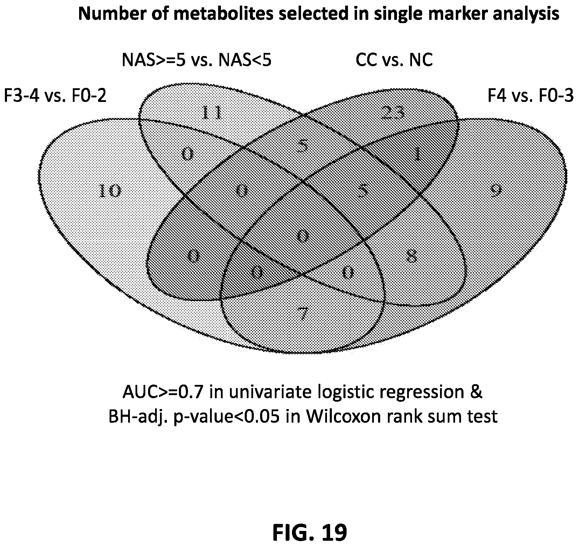

As demonstrated in Example 6 and FIG. 19, different panels of biomarkers can differentiate patients with different presentation of disease. In one disclosure, such a biomarker panel as described in Example 6 may be used to distinguish patients with the following presentations: F0-2 versus F3-4, NAS>=5 versus NAS<5, cryptogenic cirrhotics versus non-cryptogenic cirrhotics, and F4 versus F0-3 patients. Once the presentation of a patient is determined from obtaining a biological sample including the panel described in Example 6, the patient may be treated by one or more therapeutic agents as described herein.

### Monitoring of Clinical Improvements

As demonstrated in Example 1, protein markers have been identified that correlate well with the improvement of certain clinical endpoints. These protein markers, therefore, can be used to monitor the effectiveness of a treatment in a patient.

The present disclosure also discloses a method for assessing the effect of a treatment in a patient suffering from a liver disease or condition and having received the treatment. In some embodiments, the method entails measuring the expression levels of one or more proteins, selected from Tables 3A-D and 12, in a biological sample isolated from the patient; and assessing the effect of the treatment by comparing the expression levels to baseline expression levels obtained from the patients prior to the treatment.

For instance, for a patient having liver fibrosis, if the KYNU levels goes down 30% after the treatment, the patient is likely responsive to the treatment as this change suggests improvement of steatosis. By contrast, if the decrease of KYNU level is only about 5%, this patient is then likely not responding to the treatment, and a new treatment option (e.g., different drug, longer treatment or higher dose) is warranted.

Each of the clinical endpoints/variables (steatosis, lobular inflammation, hepatic ballooning, and CRN fibrosis stage) has a corresponding list of protein markers for monitoring its improvement. When the clinical endpoint is improvement of steatosis, the protein marker is selected from Table 3A, Table 4A or Table 12. When the clinical endpoint is improvement of lobular inflammation, the protein marker is selected from Table 3B, Table 4B or Table 12. When the clinical endpoint is improvement of hepatic ballooning, the protein marker is selected from Table 3C, Table 4C or Table 12. When the clinical endpoint is improvement of CRN fibrosis stage, the protein marker is selected from Table 3D or Table 4D.

Multivariate marker groups are also identified for each clinical endpoint, which are summarized in Table B. When the clinical endpoint is improvement of CRN fibrosis stage, the protein markers are two, three, four, five, six or more, or all seven selected from pTEN (P60484), CD70 (P32970), Caspase-2 (P42575), Cathepsin H (P09668), LAG-1 (Q8NHW4), PDXK (O00764), and GITR (Q9Y5U5).

When the clinical endpoint is improvement of steatosis, the protein markers are two, three, four, five, six, seven, eight, nine, ten, eleven or more, or all twelve selected from Integrin a1b1 (P56199, P05556), Nectin-like protein 2 (Q9BY67), PDGF Rb (P09619), LRP8 (Q14114), CD30 Ligand (P32971), Lumican (P51884), SAP (P02743), YKL-40 (P36222), sTie-2 (Q02763), HSP 90a/b (P07900 P08238), TSP2 (P35442), and YES (P07947).

When the clinical endpoint is improvement of lobular inflammation, the protein markers are two, three, four, five or more, or all five selected from HSP 90a/b (P07900 P08238), Aminoacylase-1 (Q03154), FCG3B (075015), M-CSF R (P07333), and Keratin 18 (P05783).

When the clinical endpoint is improvement of hepatic ballooning, the protein markers are two, three, four, five, six or more, or all seven selected from Fibronectin (P02751), Thyroxine-Binding Globulin (P05543), FGF23 (Q9GZV9), LG3BP (Q08380), Heparin cofactor II (P05546), Protein C (P04070) and STAT3 (P40763).

### Kits and Panels

The present disclosure also provides kits, packages and diagnostic panels for use in the method of the invention. For instance, the kits may include antibodies, nucleotide probes or primers and other reagents for measuring the protein or mRNA expression of various lists of proteins (e.g., Tables 1A-1F, 2A-2F, 3A-3D, 4A-4D, 11A-11D, 12, A, and B) as disclosed herein. In another embodiment, the kit or package further includes a suitable therapy.

### Panel Testing

Various embodiments disclosed above include multiple protein markers, the measurement of which can be conducted together (simultaneously or sequentially). Such testing will provide information for suitable diagnosis, prognosis, and clinical monitoring, without limitation.

A method is disclosed for providing biological information for diagnosing a liver disease or condition in a human subject, comprising measuring the expression levels of two, three, four, five, six, seven, eight, nine, ten, fifty, twenty or more proteins, selected from Tables 1A-1F, Tables 2A-2F or Table 11A-11D, in a biological sample isolated from the human subject. In some embodiments, the proteins are selected from Complement component 7 (C7), Collectin Kidney 1 (CL-K1), Insulin-like growth factor binding protein 7 (IGFBP7), Spondin-1(RSPO1), Interleukin 5 receptor subunit alpha (IL5-Ra), Matrix metallopeptidase (MMP-7), and Thrombospondin-2 (TSP2). In some disclosures, the measurement is carried out for no more than 20, 25, 30, 35, 40, or 50 proteins.

Another method is disclosed for providing biological information for determining the CRN (Nonalcoholic Steatohepatitis Clinical Research Network) fibrosis stage in a human subject, comprising measuring the expression levels of two, three, four, five, six, seven, eight, nine, ten, fifty, twenty or more proteins, selected from Tables 1A, 2A or 11A, in a biological sample isolated from the human subject. In some disclosures, the measurement is carried out for no more than 20, 25, 30, 35, 40, or 50 proteins.

Another method is disclosed for providing biological information for determining the Ishak fibrosis stage in a human subject, comprising measuring the expression levels of two, three, four, five, six, seven, eight, nine, ten, fifty, twenty or more proteins, selected from Tables 1B or 2B, in a biological sample isolated from the human subject. In some disclosures, the measurement is carried out for no more than 20, 25, 30, 35, 40, or 50 proteins.

Another method is disclosed for providing biological information for determining the NAS (nonalcoholic fatty liver disease (NAFLD) activity score) in a human subject, comprising measuring the expression levels of two, three, four, five, six, seven, eight, nine, ten, fifty, twenty or more proteins, selected from Tables 1C, 2C or 11B, in a biological sample isolated from the human subject. In some disclosures, the measurement is carried out for no more than 20, 25, 30, 35, 40, or 50 proteins.

Another method is disclosed for providing biological information for characterizing steatosis in a human subject, comprising measuring the expression levels of two, three, four, five, six, seven, eight, nine, ten, fifty, twenty or more proteins, selected from Tables 1D or 2D, in a biological sample isolated from the human subject. In some disclosures, the measurement is carried out for no more than 20, 25, 30, 35, 40, or 50 proteins.

Another method is disclosed for providing biological information for characterizing lobular inflammation in a human subject, comprising measuring the expression levels of two, three, four, five, six, seven, eight, nine, ten, fifty, twenty or more proteins, selected from Tables 1E, 2E or 11C, in a biological sample isolated from the human subject. In some disclosures, the measurement is carried out for no more than 20, 25, 30, 35, 40, or 50 proteins.

Another method is disclosed for providing biological information for characterizing hepatic ballooning in a human subject, comprising measuring the expression levels of two, three, four, five, six, seven, eight, nine, ten, fifty, twenty or more proteins, selected from Tables 1F, 2F or 11D, in a biological sample isolated from the human subject. In some disclosures, the measurement is carried out for no more than 20, 25, 30, 35, 40, or 50 proteins.

In some disclosures, the method further comprises making a diagnosis based on the biological information. In some disclosures, the method further comprises prescribing or administering to the human subject a therapy according to the diagnosis.

One disclosure provides a method for providing biological information for assessing the effect of a treatment in a patient suffering from liver disease or condition and having received the treatment, comprising measuring the expression levels of two, three, four, five, six, seven, eight, nine, ten, fifty, twenty or more proteins, selected from Tables 3A-3D and 12, in a biological sample isolated from the patient. In some disclosures, the proteins are selected from Phosphatase and tensin homolog (PTEN), CD70, Caspase 2, Cathepsin H (CTSH), Sphingosine N-acyltransferase (LAG-1), Pyridoxal kinase (PDXK), and Glucocorticoid-induced TNFR-related protein (GITR). In some disclosures, the measurement is carried out for no more than 20, 25, 30, 35, 40, or 50 proteins.

Another disclosure provides a method for providing biological information for assessing whether a liver disease or condition patient exhibits improvement on steatosis following a treatment, comprising measuring the expression levels of two, three, four, five, six, seven, eight, nine, ten, fifty, twenty or more proteins, selected from Tables 3A, 4A or 12, in a biological sample isolated from the human subject. In some disclosures, the proteins are selected from Integrin a1b1 (P56199, P05556), Nectin-like protein 2 (Q9BY67), PDGF Rb (P09619), LRP8 (Q14114), CD30 Ligand (P32971), Lumican (P51884), SAP (P02743), YKL-40 (P36222), sTie-2 (Q02763), HSP 90a/b (P07900 P08238), TSP2 (P35442), and YES (P07947). In some disclosures, the measurement is carried out for no more than 20, 25, 30, 35, 40, or 50 proteins.

Another disclosure provides a method for providing biological information for assessing whether a liver disease or condition patient exhibits improvement on lobular inflammation following a treatment, comprising measuring the expression levels of two, three, four, five, six, seven, eight, nine, ten, fifty, twenty or more proteins, selected from Tables 3B, 4B or 12, in a biological sample isolated from the human subject. In some disclosures, the proteins are selected from HSP 90a/b (P07900 P08238), Aminoacylase-1 (Q03154), FCG3B (075015), M-CSF R (P07333), and Keratin 18 (P05783). In some disclosures, the measurement is carried out for no more than 20, 25, 30, 35, 40, or 50 proteins.

Another disclosure provides a method for providing biological information for assessing whether a liver disease or condition patient exhibits improvement on hepatic ballooning following a treatment, comprising measuring the expression levels of two, three, four, five, six, seven, eight, nine, ten, fifty, twenty or more proteins, selected from Tables 3C, 4C or 12, in a biological sample isolated from the human subject. In some disclosures, the proteins are selected from Fibronectin (P02751), Thyroxine-Binding Globulin (P05543), FGF23 (Q9GZV9), LG3BP (Q08380), Heparin cofactor II (P05546), Protein C (P04070) and STAT3 (P40763). In some disclosures, the measurement is carried out for no more than 20, 25, 30, 35, 40, or 50 proteins.

Another disclosure provides a method for providing biological information for assessing whether a liver disease or condition patient exhibits improvement on CRN fibrosis stage following a treatment, comprising measuring the expression levels of two, three, four, five, six, seven, eight, nine, ten, fifty, twenty or more proteins, selected from Tables 3D or 4D, in a biological sample isolated from the human subject. In some disclosures, the proteins are selected from pTEN (P60484), CD70 (P32970), Caspase-2 (P42575), Cathepsin H (P09668), LAG-1 (Q8NHW4), PDXK (O00764), and GITR (Q9Y5U5). In some disclosures, the measurement is carried out for no more than 20, 25, 30, 35, 40, or 50 proteins.

In some disclosures, the method further comprises making a treatment assessment based on the biological information. In some disclosures, the method further comprises prescribing or administering to the human subject a therapy according to the diagnosis.

### Treatment Methods and Uses

Upon obtaining information relating to the diagnosis of a liver disease or condition, or confirmation of effectiveness of a treatment, the present disclosure further provides suitable treatment methods or uses to the patient. In some disclosures, the patient has been analyzed accordingly using any embodiment of the present disclosure, with one or more protein markers, optionally with other markers or clinical tests. In some disclosures, the treatment uses one or more of the following therapeutic agents.

### Therapeutic Agents

In some disclosures, one or more therapeutic agents include, and are not limited to, a compound disclosed herein which is administered in combination with one or more additional therapeutic agents to treat or prevent a disease or condition disclosed herein. In some disclosures, the one or more additional therapeutic agents are a(n) ACE inhibitor, Acetyl CoA carboxylase inhibitor, Adenosine A3 receptor agonist, Adiponectin receptor agonist, AKT protein kinase inhibitor, AMP-activated protein kinases (AMPK), Amylin receptor agonist, Angiotensin II AT-1 receptor antagonist, Autotaxin inhibitors, Bioactive lipid, Calcitonin agonist, Caspase inhibitor, Caspase-3 stimulator, Cathepsin inhibitor, Caveolin 1 inhibitor, CCR2 chemokine antagonist, CCR3 chemokine antagonist, CCR5 chemokine antagonist, Chloride channel stimulator, CNR1 inhibitor, Cyclin D1 inhibitor, Cytochrome P450 7A1 inhibitor, DGAT1/2 inhibitor, Dipeptidyl peptidase IV inhibitor, Endosialin modulator, Eotaxin ligand inhibitor, Extracellular matrix protein modulator, Farnesoid X receptor agonist, Fatty acid synthase inhibitors, FGF1 receptor agonist, Fibroblast growth factor (FGF-15, FGF-19, FGF-21) ligands, Galectin-3 inhibitor, Glucagon receptor agonist, Glucagon-like peptide 1 agonist, G-protein coupled bile acid receptor 1 agonist, Hedgehog (Hh) modulator, Hepatitis C virus NS3 protease inhibitor, Hepatocyte nuclear factor 4 alpha modulator (HNF4A), Hepatocyte growth factor modulator, HMG CoA reductase inhibitor, IL-10 agonist, IL-17 antagonist, Ileal sodium bile acid cotransporter inhibitor, Insulin sensitizer, integrin modulator, intereukin-1 receptor-associated kinase 4 (IRAK4) inhibitor, Jak2 tyrosine kinase inhibitor, Klotho beta stimulator, ketohexokinase inhibitors such as PF-06835919, 5-Lipoxygenase inhibitor, Lipoprotein lipase inhibitor, Liver X receptor, LPL gene stimulator, Lysophosphatidate-1 receptor antagonist, Lysyl oxidase homolog 2 inhibitor, Matrix metalloproteinases (MMPs) inhibitor, MEKK-5 protein kinase inhibitor, Membrane copper amine oxidase (VAP-1) inhibitor, Methionine aminopeptidase-2 inhibitor, Methyl CpG binding protein 2 modulator, MicroRNA-21(miR-21) inhibitor, Mitochondrial uncoupler such as nitazoxanide, Myelin basic protein stimulator, NACHT LRR PYD domain protein 3 (NLRP3) inhibitor, NAD-dependent deacetylase sirtuin stimulator, NADPH oxidase inhibitor (NOX), Nicotinic acid receptor 1 agonist, P2Y13 purinoceptor stimulator, PDE 3 inhibitor, PDE 4 inhibitor, PDE 5 inhibitor, PDGF receptor beta modulator, Phospholipase C inhibitor, PPAR alpha agonist, PPAR delta agonist, PPAR gamma agonist, PPAR gamma modulator, Protease-activated receptor-2 antagonist, Protein kinase modulator, Rho associated protein kinase inhibitor, Sodium glucose transporter-2 inhibitor, SREBP transcription factor inhibitor, STAT-1 inhibitor, Stearoyl CoA desaturase-1 inhibitor, Suppressor of cytokine signalling-1 stimulator, Suppressor of cytokine signalling-3 stimulator, Transforming growth factor β (TGF-β), Transforming growth factor β activated Kinase 1 (TAK1), Thyroid hormone receptor beta agonist, TLR-4 antagonist, Transglutaminase inhibitor, Tyrosine kinase receptor modulator, GPCR modulator, nuclear hormone receptor modulator, WNT modulators, or YAP/TAZ modulator.

Non-limiting examples of the one or more additional therapeutic agents include:
ACE inhibitors, such as enalapril;
Acetyl CoA carboxylase (ACC) inhibitors, such as DRM-01, gemcabene, PF-05175157, and QLT-091382;
Adenosine receptor agonists, such as CF-102, CF-101, CF-502, and CGS21680;
Adiponectin receptor agonists, such as ADP-355;
Amylin/calcitonin receptor agonists, such as KBP-042;
AMP activated protein kinase stimulators, such as O-304;
Angiotensin II AT-1 receptor antagonists, such as irbesartan;
Autotaxin inhibitors, such as PAT-505, PAT-048, GLPG-1690, X-165, PF-8380, and AM-063;
Bioactive lipids, such as DS-102;
Cannabinoid receptor type 1 (CNR1) inhibitors, such as namacizumab and GWP-42004;
Caspase inhibitors, such as emricasan;
Pan cathepsin B inhibitors, such as VBY-376;
Pan cathepsin inhibitors, such as VBY-825;
CCR2/CCR5 chemokine antagonists, such as cenicriviroc;
CCR2 chemokine antagonists, such as propagermanium;
CCR3 chemokine antagonists, such as bertilimumab;
Chloride channel stimulators, such as cobiprostone;
Diglyceride acyltransferase 2 (DGAT2) inhibitors, such as IONIS-DGAT2Rx;
Dipeptidyl peptidase IV inhibitors, such as linagliptin;
Eotaxin ligand inhibitors, such as bertilimumab;
Extracellular matrix protein modulators, such as CNX-024;
Fatty acid synthase inhibitors, such as TVB-2640;
Fibroblast growth factor 19 (rhFGF19)/cytochrome P450 (CYP)7A1 inhibitors, such as NGM-282;
Fibroblast growth factor 21(FGF-21) ligand, such as BMS-986171, BMS-986036;
Fibroblast growth factor 21(FGF-21)/glucagon like peptide 1 (GLP-1) agonists, such as YH-25723;
Galectin-3 inhibitors, such as GR-MD-02;
Glucagon-like peptide 1(GLP1R) agonists, such as AC-3174, liraglutide, semaglutide;
G-protein coupled bile acid receptor 1(TGR5) agonists, such as RDX-009, INT-777;
Heat shock protein 47 (HSP47) inhibitors, such as ND-L02-s0201;
HMG CoA reductase inhibitors, such as atorvastatin, fluvastatin, pitavastatin, pravastatin, rosuvastatin, and simvastatin;
IL-10 agonists, such as peg-ilodecakin;
Ileal sodium bile acid cotransporter inhibitors, such as A-4250, volixibat potassium ethanolate hydrate (SHP-262), and GSK2330672;
Insulin sensitizers, such as, KBP-042, MSDC-0602K, Px-102, RG-125 (AZD4076), and VVP-100X;
beta Klotho (KLB)- FGF1c agonist, such as NGM-313;
ketohexokinase inhibitors such as PF-06835919;
5-Lipoxygenase inhibitors, such as tipelukast (MN-001);
Lipoprotein lipase inhibitors, such as CAT-2003;
LPL gene stimulators, such as alipogene tiparvovec;
Liver X receptor (LXR) modulators, such as PX-L603, PX-L493, BMS-852927, T-0901317, GW-3965, and SR-9238;
Lysophosphatidate-1 receptor antagonists, such as BMT-053011, UD-009. AR-479, ITMN-10534, BMS-986020, and KI-16198;
Lysyl oxidase homolog 2 inhibitors, such as simtuzumab;
Semicarbazide-Sensitive Amine Oxidase/Vascular Adhesion Protein-1 (SSAO/VAP-1) Inhibitors, such as PXS-4728A;
Methionine aminopeptidase-2 inhibitors, such as ZGN-839;
Methyl CpG binding protein 2 modulators, such as mercaptamine;
Mitochondrial uncouplers, such as 2,4-dinitrophenol or nitazoxanide;
Myelin basic protein stimulators, such as olesoxime;
NADPH oxidase 1/4 inhibitors, such as GKT-831;
Nicotinic acid receptor 1 agonists, such as ARI-3037MO;
NACHT LRR PYD domain protein 3 (NLRP3) inhibitors, such as KDDF-201406-03, and NBC-6;
Nuclear receptor modulators, such as DUR-928;
P2Y13 purinoceptor stimulators, such as CER-209;
PDE 3/4 inhibitors, such as tipelukast (MN-001);
PDE 5 inhibitors, such as sildenafil;
PDGF receptor beta modulators, such as BOT-191, BOT-509;
PPAR agonists, such as elafibranor (GFT-505), MBX-8025, deuterated pioglitazone R-enantiomer, pioglitazone, DRX-065, saroglitazar, and IVA-337;
Protease-activated receptor-2 antagonists, such as PZ-235;
Protein kinase modulators, such as CNX-014;
Rho associated protein kinase (ROCK) inhibitors, such as KD-025;
Sodium glucose transporter-2(SGLT2) inhibitors, such as ipragliflozin, remogliflozin etabonate, ertugliflozin, dapagliflozin, and sotagliflozin;
SREBP transcription factor inhibitors, such as CAT-2003 and MDV-4463;
Stearoyl CoA desaturase-1 inhibitors, such as aramchol;
Thyroid hormone receptor beta agonists, such as MGL-3196, MGL-3745, VK-2809;
TLR-4 antagonists, such as JKB-121;
Tyrosine kinase receptor modulators, such as CNX-025;
GPCR modulators, such as CNX-023; and
Nuclear hormone receptor modulators, such as Px-102.

In certain specific disclosures, the one or more additional therapeutic agents are selected from A-4250, AC-3174, acetylsalicylic acid, AK-20, AKN-083, alipogene tiparvovec, aramchol, ARI-3037MO, ASP-8232, atorvastatin, bertilimumab, Betaine anhydrous, BAR-704, BI-1467335, BMS-986036, BMS-986171, BMT-053011, BOT-191, BTT-1023, BWD-100, BWL-200, CAT-2003, cenicriviroc, CER-209, CF-102, CGS21680, CNX-014, CNX-023, CNX-024, CNX-025, cobiprostone, colesevelam, dapagliflozin, 16-dehydro-pregnenolone, deuterated pioglitazone R-enantiomer, 2,4-dinitrophenol, DRX-065, DS-102, DUR-928, EDP-305, elafibranor (GFT-505), emricasan, enalapril,EP-024297, ertugliflozin, evogliptin, EYP-001, F-351, fexaramine, GKT-831, GNF-5120, GR-MD-02, hydrochlorothiazide, icosapent ethyl ester, IMM-124-E, INT-767, IONIS-DGAT2Rx, INV-33, ipragliflozin, Irbesarta, propagermanium, IVA-337, JKB-121, KB-GE-001, KBP-042, KD-025, M790, M780, M450, metformin, sildenafil, LC-280126, linagliptin, liraglutide, LJN-452, LMB-763, MBX-8025, MDV-4463, mercaptamine, MET-409, MGL-3196, MGL-3745, MSDC-0602K, namacizumab, NC-101, ND-L02-s0201, NFX-21, NGM-282, NGM-313, NGM-386, NGM-395, NTX-023-1, norursodeoxycholic acid, O-304, obeticholic acid, 25HC3S, olesoxime, PAT-505, PAT-048, peg-ilodecakin, pioglitazone, pirfenidone, PRI-724, PX20606, Px-102, PX-L603, PX-L493, PXS-4728A, PZ-235, RDX-009, RDX-023, remogliflozin etabonate, repurposed tricaprilin, RG-125 (AZD4076), saroglitazar, semaglutide, simtuzumab, SIPI-7623, solithromycin, sotagliflozin, statins (atorvastatin, fluvastatin, pitavastatin, pravastatin, rosuvastatin, simvastatin), TCM-606F, TERN-101, TEV-45478, tipelukast (MN-001), TLY-012, tropifexor, TRX-318, TVB-2640, UD-009, ursodeoxycholic acid, VBY-376, VBY-825, VK-2809, vismodegib, volixibat potassium ethanolate hydrate (SHP-626), VVP-100X, WAV-301, WNT-974, and ZGN-839

In some disclosures, the one or more therapeutic agent is an ACC inhibitor described in WO2013/071169. In some disclosures, the one or more therapeutic agent is an ASK1 inhibitor described in WO2013/112741. In some disclosures, the one or more therapeutic agent is an FXR agonist such as the one described in WO2013/007387. In particular disclosures, the two therapeutic agents are an ASK1 and an ACC inhibitor. In particular disclosures, the therapeutic agents are an FXR agonist and an ASK1 inhibitor. In still other disclosures, the two therapeutic agents are an FXR agonist and an ACC inhibitor. In yet another disclosure, three therapeutic agents are used: an ASK1 inhibitor, and ACC inhibitor, and an FXR agonist.

### Pharmaceutical Compositions and Modes of Administration

Compounds provided herein are usually administered in the form of pharmaceutical compositions. Thus, provided herein are also pharmaceutical compositions that contain one or more of the compounds described herein or a pharmaceutically acceptable salt, tautomer, stereoisomer, mixture of stereoisomers, prodrug, or deuterated analog thereof and one or more pharmaceutically acceptable vehicles selected from carriers, adjuvants and excipients. Suitable pharmaceutically acceptable vehicles may include, for example, inert solid diluents and fillers, diluents, including sterile aqueous solution and various organic solvents, permeation enhancers, solubilizers and adjuvants. Such compositions are prepared in a manner well known in the pharmaceutical art. *See*, *e.g*., Remington's Pharmaceutical Sciences, Mace Publishing Co., Philadelphia, Pa. 17th Ed. (1985); and Modern Pharmaceutics, Marcel Dekker, Inc. 3rd Ed. (G.S. Banker & C.T. Rhodes, Eds.).

The pharmaceutical compositions may be administered in either single or multiple doses. The pharmaceutical composition may be administered by various methods including, for example, rectal, buccal, intranasal and transdermal routes. In certain embodiments, the pharmaceutical composition may be administered by intra-arterial injection, intravenously, intraperitoneally, parenterally, intramuscularly, subcutaneously, orally, topically, or as an inhalant.

One mode for administration is parenteral, for example, by injection. The forms in which the pharmaceutical compositions described herein may be incorporated for administration by injection include, for example, aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Oral administration may be another route for administration of the compounds described herein. Administration may be via, for example, capsule or enteric coated tablets. In making the pharmaceutical compositions that include at least one compound described herein or a pharmaceutically acceptable salt, tautomer, stereoisomer, mixture of stereoisomers, prodrug, or deuterated analog thereof, the active ingredient is usually diluted by an excipient and/or enclosed within such a carrier that can be in the form of a capsule, sachet, paper or other container. When the excipient serves as a diluent, it can be in the form of a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include lubricating agents such as talc, magnesium stearate, and mineral oil; wetting agents; emulsifying and suspending agents; preserving agents such as methyl and propylhydroxy-benzoates; sweetening agents; and flavoring agents.

The compositions that include at least one compound described herein or a pharmaceutically acceptable salt, tautomer, stereoisomer, mixture of stereoisomers, prodrug, or deuterated analog thereof can be formulated so as to provide quick, sustained or delayed release of the active ingredient after administration to the subject by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Patent Nos. 3,845,770; 4,326,525; 4,902,514; and 5,616,345. Another formulation for use in the methods disclosed herein employ transdermal delivery devices ("patches"). Such transdermal patches may be used to provide continuous or discontinuous infusion of the compounds described herein in controlled amounts. The construction and use of transdermal patches for the delivery of pharmaceutical agents is well known in the art. *See*, *e.g*., U.S. Patent Nos. 5,023,252, 4,992,445 and 5,001,139. Such patches may be constructed for continuous, pulsatile, or on demand delivery of pharmaceutical agents.

For preparing solid compositions such as tablets, the principal active ingredient may be mixed with a pharmaceutical excipient to form a solid preformulation composition containing a homogeneous mixture of a compound described herein or a pharmaceutically acceptable salt, tautomer, stereoisomer, mixture of stereoisomers, prodrug, or deuterated analog thereof. When referring to these preformulation compositions as homogeneous, the active ingredient may be dispersed evenly throughout the composition so that the composition may be readily subdivided into equally effective unit dosage forms such as tablets, pills and capsules.

The tablets or pills of the compounds described herein may be coated or otherwise compounded to provide a dosage form affording the advantage of prolonged action, or to protect from the acid conditions of the stomach. For example, the tablet or pill can include an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation may include solutions and suspensions in pharmaceutically acceptable, aqueous or organic solvents, or mixtures thereof, and powders. The liquid or solid compositions may contain suitable pharmaceutically acceptable excipients as described herein. In some embodiments, the compositions are administered by the oral or nasal respiratory route for local or systemic effect. In other embodiments, compositions in pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

### EXAMPLES

The following examples are included to demonstrate specific embodiments of the disclosure. It should be appreciated by those of skill in the art that the techniques disclosed in the examples which follow represent techniques to function well in the practice of the disclosure, and thus can be considered to constitute specific modes for its practice. However, those of skill in the art should, in light of the present disclosure, appreciate that many changes can be made in the specific embodiments which are disclosed and still obtain a like or similar result without departing from the spirit and scope of the disclosure.

### Example 1: Evaluation of SOMAscan as a discovery platform to identify non-invasive protein biomarkers in NASH patients treated with selonsertib

This example employed SOMAscan to identify candidate protein biomarkers for diagnosis and disease monitoring in F2-F3 NASH subjects. SOMAscan is a proteomic biomarker discovery platform and has been used to identify disease-associated protein biomarkers in blood and other biological fluids.

### Method:

Seventy-two subjects with nonalcoholic steatohepatitis (NASH) (NAS ≥ 5) and F2-3 fibrosis were treated with selonsertib (SEL, an inhibitor of apoptosis signal-regulating kinase 1 (ASK1)) 6 mg or 18 mg orally QD alone or in combination with simtuzumab (SIM, 125 mg SQ weekly) or SIM alone for 24 weeks. Baseline and week 24 serum samples from these study subjects together with additional F0, F1 and F4 baseline samples were tested with SOMAscan (SOMAlogic, Inc., Boulder, CO). Associations of 1300 proteins with fibrosis stages and NAS components at baseline and after 24 weeks were examined.

### Results:

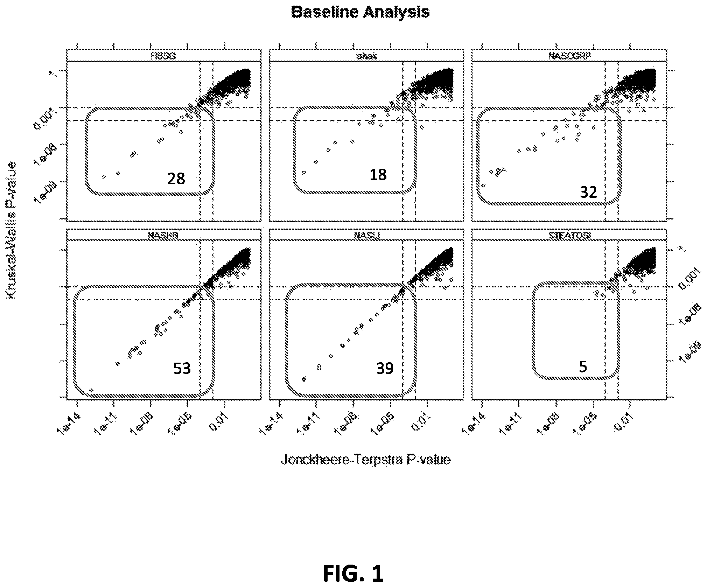

Univariate analysis identified 28 proteins that are significantly (p<0.001 for both Kruskal-Wallis test and Jonckheere-Terpstra test, only Kruskal-Wallis test p values are shown in the tables below; representative protein markers for CRN fibrosis stages are shown in **FIG. 1**) associated with CRN fibrosis stage, 18 proteins that are significantly associated with Ishak fibrosis stage, 34 proteins significantly associated with NAS score, 7 proteins that are significantly associated with steatosis, 39 proteins that are significantly associated with lobular inflammation, and 53 proteins that are significantly associated with hepatic ballooning at baseline. Among these proteins, several markers (ACY1, HSP90a/b, and Integrin a1b1) are associated with fibrosis stage, steatosis, lobular inflammation and hepatic ballooning. The univariate results are presented in Table 1A-F below (the protein expression levels are shown in Relative Fluorescent Unit (RFU), which is the readout from the instrument).

### Tables 1A-F: Protein markers for diagnosis

**Table 1A. Protein Markers for CRN Fibrosis Stages (CRN)**

| **Marker Name** | **UniProt** | **p value** | **CRN fibrosis stage 0** | **CRN fibrosis stage 1** | **CRN fibrosis stage 2** | **CRN fibrosis stage 3** | **CRN fibrosis stage 4** |
|---|---|---|---|---|---|---|---|
| Collectin Kidney 1 | Q9BWP8 | 2.21E-09 | 5987.2 | 4903.3 | 8174.95 | 11267.55 | 15604.5 |
| C7 | P10643 | 4.14E-09 | 2173.6 | 1930.4 | 2330.55 | 2590.2 | 3379.95 |
| IGFBP-7 | 0.16270 | 1.09E-07 | 5898.4 | 6361.5 | 7167.65 | 8593.2 | 9992.25 |
| MMP-7 | P09237 | 2.21E-07 | 1220.8 | 1321.9 | 1577.3 | 2013.65 | 2684.1 |
| Spondin-1 | Q9HCB6 | 1.53E-06 | 1600.8 | 1760.5 | 1825.3 | 2131.55 | 3023.9 |
| TSP2 | P35442 | 2.29E-06 | 575.9 | 879.3 | 1396.65 | 1761.05 | 1791.45 |
| sE-Selectin | P16581 | 3.18E-05 | 23557.3 | 25301.3 | 29424.7 | 36202.85 | 49398.9 |
| IL-1 sRI | P14778 | 4.52E-05 | 3858.3 | 3942.3 | 4198.55 | 5472.45 | 7327.05 |
| 6Ckine | O00585 | 4.25E-05 | 5152.7 | 4733.2 | 5371.65 | 6293.8 | 7693.5 |
| LTBP4 | Q8N2S1 | 5.35E-05 | 935.5 | 1033.7 | 1098.4 | 1210.85 | 1467 |
| MIC-1 | Q99988 | 3.48E-05 | 569.4 | 657 | 917 | 1046.05 | 1246.8 |
| XPNPEP1 | Q9NQW7 | 8.45E-05 | 2285.1 | 2688.5 | 3077.95 | 3519.55 | 4201.25 |
| IL-18 BPa | O95998 | 1.14E-05 | 6253.3 | 7763.5 | 8939.8 | 8942.1 | 12208.5 |
| SHP-2 | Q06124 | 0.00014999 | 2444.7 | 2746.1 | 3160.2 | 3676.75 | 4811.5 |
| IL-19 | Q9UHD0 | 0.00054898 | 6650.8 | 6412 | 7286.7 | 8225.55 | 9611.05 |
| ERAB | Q99714 | 0.00024364 | 915.8 | 1030.6 | 1101 | 1340.2 | 1274.2 |
| BSSP4 | Q9GZN4 | 0.00019407 | 816.3 | 900.9 | 974.95 | 1073.35 | 1316.85 |
| HSP 70 | P0DMV8 | 0.00084118 | 5665 | 6574.1 | 7380.75 | 8344.75 | 10561.55 |
| SEM6B | Q9H3T3 | 0.00013854 | 3329.4 | 4060.3 | 4452.6 | 4726.25 | 6304.8 |
| SLAF7 | Q9NQ25 | 0.00052829 | 46254.9 | 45233.4 | 48063.25 | 56140.5 | 74638.2 |
| CD48 | P09326 | 0.00075613 | 623.6 | 660 | 672.5 | 747.35 | 758.55 |
| FSTL3 | O95633 | 0.00088627 | 3254.9 | 3410 | 3497.55 | 3948.25 | 4403.1 |
| sCD163 | Q86VB7 | 0.00058386 | 2026.6 | 2190.2 | 2614.75 | 2745.6 | 3265.25 |
| NADPH-P450 Oxidoreductase | P16435 | 0.00043972 | 5941 | 6559.3 | 9070.3 | 10918.25 | 11295.15 |
| LIF sR | P42702 | 0.00084016 | 3449.2 | 3827 | 3873.75 | 4142.7 | 5350.85 |
| IL-8 | P10145 | 0.00084147 | 1456.2 | 1572.7 | 1836.55 | 1895.75 | 2405.2 |
| CD30 Ligand | P32971 | 0.0002216 | 2017.3 | 2341.4 | 2410.75 | 2459.35 | 2877.35 |
| SAP | P02743 | 0.00035833 | 34662 | 31095.8 | 29256.95 | 28337.7 | 23256.35 |

**Table 1B. Protein Markers for Ishak Fibrosis Stages (not according to the invention)**

| **Marker Name** | **UniProt** | **p value** | **Ishak fibrosis stage 0** | **Ishak fibrosis stage 1** | **Ishak fibrosis stage 2** | **Ishak fibrosis stage 3** | **Ishak fibrosis stage 4** | **Ishak fibrosis stage 5** | **Ishak fibrosis stage 6** |
|---|---|---|---|---|---|---|---|---|---|
| Collectin Kidney 1 | Q9BWP8 | 5.05E-09 | 4963.25 | 5586.5 | 8304.6 | 9376.5 | 13458.5 | 11252.75 | 24984.7 |
| C7 | P10643 | 3.44E-08 | 2040.3 | 1972.6 | 2339.6 | 2419.1 | 2837.8 | 3313.525 | 3711.1 |
| IGFBP-7 | Q16270 | 3.41E-07 | 5739.9 | 6361.5 | 7210.5 | 7783.2 | 8979 | 8668.9 | 10130.15 |
| MMP-7 | P09237 | 1.14E-06 | 1271.35 | 1304 | 1590.9 | 1904.2 | 2313.6 | 2836.075 | 2784.5 |
| TSP2 | P35442 | 1.53E-06 | 738.85 | 859.4 | 1375.9 | 1426.8 | 2288.8 | 2220.55 | 1791.45 |
| sE-Selectin | P16581 | 4.76E-05 | 22373.85 | 25498 | 29931 | 34267.9 | 41464.2 | 40645.18 | 50563 |
| LTBP4 | Q8N2S1 | 6.18E-05 | 930.3 | 1059.7 | 1102.7 | 1113.8 | 1247.7 | 1466.4 | 1501 |
| MIC-1 | Q99988 | 4.79E-05 | 561.3 | 657 | 917.9 | 936.9 | 1144.1 | 1334.4 | 1236.3 |
| IL-1 sRI | P14778 | 0.000214 | 3701.65 | 3942.3 | 4265.2 | 5393.4 | 5330.4 | 7033.1 | 6845.45 |
| 6Ckine | O00585 | 0.000257 | 4942.95 | 4852 | 5341.4 | 6155.1 | 6818.5 | 7692.525 | 7354.8 |
| Spondin-1 | Q9HCB6 | 3.82E-05 | 1595.9 | 1871.4 | 1842.2 | 2112.1 | 2123.4 | 2916.7 | 3102.6 |
| XPNPEP1 | Q9NQW7 | 0.000539 | 2282.3 | 2753.5 | 3094.8 | 3447.2 | 3868 | 4279.625 | 4090.7 |
| ERAB | Q99714 | 0.000995 | 989.1 | 1030.6 | 1105.1 | 1258.2 | 1500.6 | 1498.35 | 1345.45 |
| BSSP4 | Q9GZN4 | 0.000525 | 858.6 | 896.9 | 981.2 | 1023.6 | 1187.2 | 1443.1 | 1316.85 |
| SAP | P02743 | 0.000271 | 31665.7 | 31374.3 | 29587.8 | 30274.7 | 27366.6 | 26341.85 | 20586.05 |
| IL-19 | Q9UHD0 | 0.001486 | 6639.5 | 6646.5 | 7290.4 | 7639.5 | 8622.7 | 8096 | 10594.2 |
| IL-8 | P10145 | 0.000723 | 1456 | 1584.9 | 1742.2 | 1857 | 1962.8 | 2554.575 | 2611.3 |
| SEM6B | Q9H3T3 | 0.000799 | 3305.25 | 4083 | 4488.2 | 4487.5 | 4742.2 | 6640.3 | 6304.8 |

**Table 1C. Protein Markers for NAS Scores (NAS) (not according to the invention)**

| **Marker Name** | **UniProt** | **p value** | **NAS stages** | | | | | | | |
|---|---|---|---|---|---|---|---|---|---|---|
| Aminoacylase-1 | Q03154 | 5.29E-10 | 4603.1 | 5770.9 | 9341.9 | 10238.4 | 14257.5 | 17923.1 | 23098.7 | 19241.9 |
| Integrin a1b1 | P56199, P05556 | 3.17E-09 | 781 | 926.8 | 956.1 | 1269.5 | 1309.1 | 2006.25 | 2542.2 | 1635 |
| TSP2 | P35442 | 5.02E-09 | 603.9 | 652.1 | 901.8 | 975.3 | 1458.6 | 1773.25 | 2363.5 | 2284.4 |
| HSP 90a/b | P07900 P08238 | 5.26E-09 | 2710.5 | 3648.6 | 3606.3 | 4344.65 | 4465.1 | 5661.85 | 7165.9 | 7471.5 |
| HSP 90b | P08238 | 7.07E-09 | 11239.4 | 14527.4 | 16297.7 | 18441.65 | 18554 | 24236.45 | 27701.9 | 29499.4 |
| NADPH-P450 Oxidoreductase | P16435 | 8.42E-09 | 4256.6 | 5687.5 | 6126.2 | 7149.85 | 8088 | 11520.7 | 12706.2 | 16688.9 |
| KYNU | Q16719 | 9.53E-09 | 1596.4 | 1306.5 | 1749.9 | 1755.4 | 2097.9 | 2830.7 | 3690.8 | 3831.1 |
| ERAB | Q99714 | 2.59E-07 | 985.9 | 901.3 | 937.4 | 1032.15 | 1135.2 | 1434.65 | 1396.2 | 1497.4 |
| PHI | P06744 | 3.24E-07 | 338.2 | 503.6 | 472.7 | 575.3 | 724.1 | 789.65 | 755.2 | 1174.4 |
| FTCD | O95954 | 3.24E-07 | 2750.3 | 8873.4 | 8231.4 | 18059.9 | 21526.2 | 31366.9 | 34054.8 | 46087.7 |
| HSP 70 | P0DMV8 | 6.89E-07 | 4723.9 | 5461.8 | 6315 | 7491.25 | 7694.2 | 8903.45 | 10700.5 | 10243.7 |
| sE-Selectin | P16581 | 1.74E-06 | 16016.8 | 20281.3 | 29931 | 28256.45 | 30675.2 | 36832.25 | 41029 | 45677.7 |
| Testican-1 | Q08629 | 2.15E-06 | 11858.9 | 9319.1 | 9409.3 | 9169.7 | 7865.6 | 7660.65 | 7854.7 | 8924.2 |
| ApoM | O95445 | 2.99E-06 | 5405.9 | 3828.2 | 4477.2 | 3809.6 | 3240.1 | 3155.35 | 3195.5 | 3026.8 |
| XPNPEP1 | Q9NQW7 | 6.28E-06 | 1941.6 | 2384.2 | 2429.7 | 2932.9 | 3328.7 | 3635.1 | 4251.6 | 4766.6 |
| HEMK2 | Q9Y5N5 | 6.63E-06 | 11355.9 | 9257 | 9087.2 | 8659.15 | 7762 | 7573.85 | 7178 | 8489.3 |
| Collectin Kidney 1 | Q9BWP8 | 1.92E-05 | 4522.9 | 4689.7 | 7122.6 | 8226.4 | 8045.3 | 12168 | 16342.4 | 10018.1 |
| ENPP7 | Q6UWV6 | 2.20E-05 | 6355.4 | 2192 | 4459.6 | 6498.85 | 7703.1 | 6493.85 | 10944.8 | 12407.3 |
| AK1A1 | P14550 | 2.51E-05 | 1170 | 1582.5 | 1771.3 | 1691.95 | 1915.8 | 2408.75 | 3121.6 | 2642.9 |
| C1-Esterase Inhibitor | P05155 | 2.74E-05 | 6066.3 | 7494.9 | 8541.6 | 6895.8 | 6346.3 | 5897.9 | 6176.7 | 5234.7 |
| HSP70 protein 8 | P11142 | 3.25E-05 | 2452.1 | 2911.8 | 2661.3 | 2979.2 | 3134.4 | 3255.35 | 3466.1 | 4060.1 |
| Cytochrome c | P99999 | 9.34E-05 | 1771.5 | 1339.2 | 1412.4 | 1316.25 | 1244.5 | 1194 | 1205.1 | 1314.5 |
| C7 | P10643 | 0.000107 | 1930.4 | 2111.3 | 2267.9 | 2153.35 | 2368.8 | 2643.25 | 3093.5 | 2804.6 |
| Cathepsin D | P07339 | 0.00025 | 867.8 | 731 | 955.1 | 893.8 | 1054.5 | 1200.2 | 1402.9 | 1037.7 |
| CD30 Ligand | P32971 | 0.000279 | 2209.5 | 2067.4 | 2239 | 2420.4 | 2458 | 2524.5 | 2688.7 | 2568.3 |
| YES | P07947 | 0.000323 | 1122.1 | 1187 | 1331.5 | 1359.2 | 1426.9 | 1522.05 | 1684.5 | 1692.5 |
| PIGR | P01833 | 0.000487 | 3151.5 | 2740.5 | 4116.2 | 3579.5 | 4070.7 | 4843.85 | 4827.2 | 6861.9 |
| QORL1 | O95825 | 0.000554 | 2484.4 | 2828.5 | 3099.3 | 3354.25 | 3708.7 | 3837.05 | 2982.6 | 3195.1 |
| AMPM2 | P50579 | 0.000565 | 9067.1 | 10689.6 | 14417.8 | 13313.45 | 13071.7 | 16369.35 | 16596.3 | 20628.8 |
| IL-19 | Q9UHD0 | 0.000619 | 5871.2 | 5731.1 | 7359.9 | 6945.55 | 7826.4 | 7867.15 | 10139.2 | 7308 |
| BMP-1 | P13497 | 0.000632 | 843.3 | 886.9 | 1257.2 | 1245.3 | 1141.3 | 1153.3 | 1272.7 | 1329.6 |
| CAMK2D | Q13557 | 0.000684 | 2680.4 | 2866.5 | 2892.3 | 3264.25 | 3420.5 | 3633.15 | 3776.1 | 4058.2 |
| sCD163 | Q86VB7 | 0.000766 | 2429.2 | 1943.2 | 2362.6 | 2258.15 | 2717.5 | 2782.95 | 2917.8 | 3033 |
| M-CSF R | P07333 | 0.000838 | 321.9 | 246.8 | 294.6 | 320.35 | 361.3 | 356.35 | 486.9 | 367 |

**Table 1D. Protein Markers for Steatosis (not according to the invention)**

| **Marker Name** | **UniProt** | **p value** | **Steatosis stage 0** | **Steatosis stage 1** | **Steatosis stage 2** | **Steatosis stage 3** |
|---|---|---|---|---|---|---|
| HSP 90b | P08238 | 0.000218 | 14145.9 | 19107.9 | 22238.45 | 26074 |
| HSP 90a/b | P07900 | 0.000221 | 3373.4 | 4670.85 | 5334.6 | 6426.6 |
| | P08238 | | | | | |
| HSP 70 | P0DMV8 | 0.000354 | 5647.5 | 8043.75 | 8569.6 | 7897.6 |
| CAMK2D | Q13557 | 0.000453 | 2680.4 | 3298.3 | 3753.95 | 3728.9 |
| Integrin a1b1 | P56199, P05556 | 0.000688 | 866.5 | 1478.55 | 1602.65 | 1464.8 |
| Aminoacylase-1 | Q03154 | 0.000779 | 6993 | 13331 | 15128.35 | 18432.1 |

**Table 1E. Protein Markers for Lobular Inflammation (LI) (not according to the invention)**

| **Marker Name** | **UniProt** | **p value** | **Lobular inflammation stage 0** | **Lobular inflammation stage 1** | **Lobular inflammation stage 2** |
|---|---|---|---|---|---|
| NADPH-P450 Oxidoreductase | P16435 | 3.03E-11 | 5303.25 | 7040 | 12303.55 |
| Aminoacylase-1 | Q03154 | 2.63E-11 | 6042.6 | 11085.1 | 19724.4 |
| Integrin a1b1 | P56199, P05556 | 2.75E-11 | 869.1 | 1233.25 | 2041.7 |
| KYNU | Q16719 | 2.69E-10 | 1400.15 | 1837.35 | 3042.45 |
| HSP 90b | P08238 | 3.87E-10 | 14336.65 | 17853.9 | 25489.65 |
| HSP 90a/b | P07900 P08238 | 2.36E-09 | 3646.55 | 4192.55 | 6228.25 |
| FTCD | O95954 | 1.62E-08 | 5909.3 | 14891.15 | 32845.3 |
| ERAB | Q99714 | 2.01E-08 | 911.15 | 1083 | 1386.65 |
| HSP 70 | P0DMV8 | 4.56E-08 | 5751.85 | 7117.95 | 9299.45 |
| XPNPEP1 | Q9NQW7 | 2.09E-07 | 2446.55 | 2814.9 | 3918.35 |
| PHI | P06744 | 1.73E-07 | 472.1 | 592.95 | 861.4 |
| Collectin Kidney 1 | Q9BWP8 | 3.68E-07 | 4675.05 | 7928.35 | 12412.75 |
| sE-Selectin | P16581 | 5.63E-07 | 18974.2 | 29030.55 | 38522.55 |
| TSP2 | P35442 | 7.19E-07 | 647.3 | 1112.8 | 1953.2 |
| AK1A1 | P14550 | 1.95E-06 | 1572.15 | 1784.1 | 2571.85 |
| dopa decarboxylase | P20711 | 5.65E-06 | 335.95 | 341.85 | 415.3 |
| HTRA2 | O43464 | 1.73E-05 | 4398.7 | 4940.3 | 5524.3 |
| YES | P07947 | 2.33E-05 | 1178.4 | 1335.15 | 1612.3 |
| ApoM | O95445 | 2.90E-05 | 4449.85 | 3773.2 | 3155.35 |
| AMPM2 | P50579 | 3.23E-05 | 11354.55 | 13312.3 | 16787.55 |
| IGFBP-7 | Q16270 | 3.56E-05 | 6824.55 | 7146.1 | 8682.05 |
| C1-Esterase Inhibitor | P05155 | 7.05E-05 | 7848.45 | 6795.4 | 5911.95 |
| C7 | P10643 | 0.000127 | 2111.25 | 2284.6 | 2665.55 |
| PIK3CA/PIK3R1 | P42336 | 0.000174 | 777.1 | 826.75 | 922 |
| | P27986 | | | | |
| HSP70 protein 8 | P11142 | 0.000131 | 2903.05 | 2959.65 | 3370.4 |
| PIGR | P01833 | 7.61E-05 | 2946 | 3806.8 | 4801.1 |
| CD30 Ligand | P32971 | 5.74E-05 | 2126.05 | 2395.3 | 2611.6 |
| IGFBP-5 | P24593 | 0.000147 | 1792.4 | 1881.85 | 1671.25 |
| NAGK | Q9UJ70 | 0.000234 | 816.05 | 931.05 | 1051.2 |
| PLXB2 | 015031 | 0.000303 | 2789.9 | 3417.55 | 3817.1 |
| IL-19 | Q9UHD0 | 0.000307 | 5868.8 | 7329.4 | 8338.8 |
| sCD163 | Q86VB7 | 0.000396 | 2001.7 | 2413.4 | 2846.25 |
| MDHC | P40925 | 0.0006 | 10621.8 | 12866.5 | 15239.55 |
| PLXC1 | O60486 | 0.000462 | 801.4 | 863.9 | 973.3 |
| IL-1 sRII | P27930 | 0.000441 | 7385.85 | 8902.45 | 10079.9 |
| HEMK2 | Q9Y5N5 | 0.000244 | 9389.7 | 8514.75 | 7863.05 |
| Testican-1 | Q08629 | 0.000375 | 9543.6 | 8546.45 | 7860.5 |
| NSE | P09104 | 0.000543 | 1103.55 | 1001.9 | 929 |
| Cathepsin D | P07339 | 0.00084 | 806.9 | 959.85 | 1153.2 |

**Table 1F. Protein Markers for Hepatic Ballooning (HB) (not according to the invention)**

| **Marker Name** | **UniProt** | **p value** | **Hepatic ballooning stage 0** | **Hepatic ballooning stage 1** | **Hepatic ballooning stage 2** |
|---|---|---|---|---|---|
| TSP2 | P35442 | 3.86E-12 | 767.6 | 1105.2 | 1973.2 |
| Aminoacylase-1 | Q03154 | 5.85E-10 | 9112.9 | 11498.3 | 17923.1 |
| ERAB | Q99714 | 1.85E-09 | 985.9 | 1062.8 | 1364.45 |
| Integrin a1b1 | P56199, P05556 | 4.79E-09 | 949.1 | 1291.2 | 2006.25 |
| HSP 90a/b | P07900 | 7.19E-09 | 3648.6 | 4465.1 | 5581.3 |
| | P08238 | | | | |
| NADPH-P450 Oxidoreductase | P16435 | 7.22E-09 | 5941 | 8104.5 | 11520.7 |
| HSP 90b | P08238 | 1.28E-08 | 15659.5 | 18730.6 | 23601.1 |
| C7 | P10643 | 1.07E-08 | 2100.3 | 2173.8 | 2706.1 |
| Collectin Kidney 1 | Q9BWP8 | 3.82E-09 | 5987.2 | 8714.3 | 11197.75 |
| KYNU | Q16719 | 2.94E-08 | 1596.4 | 1796.6 | 2883.4 |
| PHI | P06744 | 3.31E-08 | 472.7 | 577.1 | 789.65 |
| XPNPEP1 | Q9NQW7 | 1.26E-07 | 2404.7 | 3127.6 | 3706.4 |
| Cathepsin D | P07339 | 5.34E-07 | 778.3 | 924.5 | 1214.15 |
| FTCD | O95954 | 3.47E-07 | 8873.4 | 16470.2 | 30715.2 |
| HSP 70 | P0DMV8 | 6.08E-07 | 6129.5 | 7057.1 | 9118.35 |
| AK1A1 | P14550 | 2.99E-07 | 1582.5 | 1757.3 | 2286.8 |
| sCD163 | Q86VB7 | 1.68E-07 | 1966.1 | 2472.6 | 2863.15 |
| HEMK2 | Q9Y5N5 | 8.82E-07 | 9257 | 8618.6 | 7700.3 |
| ApoM | O95445 | 2.58E-07 | 4477.2 | 3640 | 3174.15 |
| sE-Selectin | P16581 | 1.23E-06 | 25015.8 | 29949.5 | 36832.25 |
| Testican-1 | Q08629 | 2.69E-06 | 9409.3 | 8911.2 | 7820.55 |
| HSP70 protein 8 | P11142 | 9.96E-06 | 2844.1 | 2991.3 | 3343 |
| Cytochrome c | P99999 | 9.56E-06 | 1369.4 | 1317.6 | 1205.7 |
| SHP-2 | Q06124 | 8.69E-06 | 2489.8 | 2969.1 | 3735.45 |
| CD30 Ligand | P32971 | 3.86E-06 | 2123.2 | 2429.3 | 2611.6 |
| C1-Esterase Inhibitor | P05155 | 2.92E-05 | 7494.9 | 6866.2 | 6074.35 |
| YES | P07947 | 3.45E-05 | 1219.3 | 1348.4 | 1569 |
| PIGR | P01833 | 3.58E-05 | 3612.4 | 3694.2 | 4973.5 |
| PLXC1 | O60486 | 3.54E-05 | 801.1 | 898.8 | 964.3 |
| IL-1 sRI | P14778 | 6.90E-05 | 3581.3 | 4558.4 | 5651.75 |
| M-CSF R | P07333 | 7.07E-05 | 283.4 | 324.8 | 371.55 |
| CAMK2D | Q13557 | 5.51E-05 | 2931.9 | 3127.5 | 3711.65 |
| FABPL | P07148 | 9.97E-05 | 4770.5 | 5401.5 | 6909.6 |
| HSP 60 | P10809 | 0.000107 | 1757 | 1693.8 | 1972.3 |
| IGFBP-7 | Q16270 | 0.000172 | 6612.2 | 7445 | 8525.65 |
| Gelsolin | P06396 | 0.000172 | 553.7 | 520.1 | 464.05 |
| CD48 | P09326 | 0.000204 | 657.9 | 688.7 | 753.05 |
| PSA | P07288 | 0.000237 | 1664.7 | 1435.4 | 1245 |
| IGFBP-5 | P24593 | 0.000229 | 1999.2 | 1787 | 1700.5 |
| MMP-7 | P09237 | 0.00022 | 1365.3 | 1604.2 | 2048.9 |
| IL-19 | Q9UHD0 | 0.000303 | 6650.8 | 7034.5 | 8107.95 |
| AMPM2 | P50579 | 0.000336 | 12818.4 | 13425.9 | 16362.15 |
| Nectin-like protein 2 | Q9BY67 | 0.000463 | 1700.5 | 1818.9 | 1997.55 |
| Glutamate carboxypeptidase | Q96KP4 | 0.000262 | 4745.6 | 4343 | 4036.1 |
| CATZ | Q9UBR2 | 0.000709 | 4278.7 | 4655.3 | 5183.05 |
| ENPP7 | Q6UWV6 | 0.000121 | 3489.3 | 6355.8 | 7882.05 |
| PLXB2 | 015031 | 0.000752 | 3023.5 | 3310.8 | 3786 |
| IL-18 BPa | O95998 | 0.000771 | 7737.2 | 8266.1 | 9431.35 |
| dopa decarboxylase | P20711 | 0.000963 | 337.7 | 366.2 | 404.55 |
| STAT1 | P42224 | 0.000756 | 713.3 | 800.6 | 918.45 |
| FSTL3 | O95633 | 0.000891 | 3377.9 | 3393.1 | 4021.05 |
| IL-18 Ra | Q13478 | 0.00034 | 1010.8 | 965.4 | 1109.1 |
| IL-1 R AcP | Q9NPH3 | 0.00022 | 16533.8 | 13461.7 | 12779.85 |

The markers in Tables 1A-F were further manually filtered with their activities and other information and the filtered lists are presented in Tables 2A-F below.

### Tables 2A-F: Protein markers for diagnosis, filtered

**Table 2A. Protein Markers for CRN Fibrosis Stages, Filtered**

| **Marker Name** | **UniProt** |
|---|---|
| MMP-7 | P09237 |
| Spondin-1 | Q9HCB6 |
| sE-Selectin | P16581 |
| IL-1 sRI | P14778 |
| 6Ckine | O00585 |
| LTBP4 | Q8N2S1 |
| MIC-1 | Q99988 |
| XPNPEP1 | Q9NQW7 |
| IL-18 BPa | O95998 |
| SHP-2 | Q06124 |
| IL-19 | Q9UHD0 |
| ERAB | Q99714 |
| BSSP4 | Q9GZN4 |
| HSP 70 | P0DMV8 |
| SEM6B | Q9H3T3 |
| SLAF7 | Q9NQ25 |
| CD48 | P09326 |
| FSTL3 | O95633 |
| sCD163 | Q86VB7 |
| LIF sR | P42702 |
| IL-8 | P10145 |
| CD30 Ligand | P32971 |
| SAP | P02743 |

**Table 2B. Protein Markers for Ishak Fibrosis Stages, Filtered (not according to the invention)**

| **Marker Name** | **UniProt** |
|---|---|
| MMP-7 | P09237 |
| sE-Selectin | P16581 |
| LTBP4 | Q8N2S1 |
| MIC-1 | Q99988 |
| IL-1 sRI | P14778 |
| 6Ckine | O00585 |
| Spondin-1 | Q9HCB6 |
| XPNPEP1 | Q9NQW7 |
| ERAB | Q99714 |
| BSSP4 | Q9GZN4 |
| SAP | P02743 |
| IL-19 | Q9UHD0 |
| IL-8 | P10145 |
| SEM6B | Q9H3T3 |

**Table 2C. Protein Markers for NAS Scores, Filtered (not according to the invention)**

| **Marker Name** | **UniProt** |
|---|---|
| HSP 90b | P08238 |
| ERAB | Q99714 |
| PHI | P06744 |
| FTCD | O95954 |
| HSP 70 | P0DMV8 |
| sE-Selectin | P16581 |
| Testican-1 | Q08629 |
| ApoM | O95445 |
| XPNPEP1 | Q9NQW7 |
| HEMK2 | Q9Y5N5 |
| ENPP7 | Q6UWV6 |
| AK1A1 | P14550 |
| C1-Esterase Inhibitor | P05155 |
| HSP70 protein 8 | P11142 |
| Cytochrome c | P99999 |
| Cathepsin D | P07339 |
| CD30 Ligand | P32971 |
| YES | P07947 |
| PIGR | P01833 |
| QORL1 | O95825 |
| AMPM2 | P50579 |
| IL-19 | Q9UHD0 |
| BMP-1 | P13497 |
| CAMK2D | Q13557 |
| sCD163 | Q86VB7 |

**Table 2D. Protein Markers for Steatosis, Filtered (not according to the invention)**

| **Marker Name** | **UniProt** |
|---|---|
| HSP 70 | P0DMV8 |
| CAMK2D | Q13557 |

**Table 2E. Protein Markers for Lobular Inflammation, Filtered (not according to the invention)**

| **Marker Name** | **UniProt** |
|---|---|
| HSP 90b | P08238 |
| FTCD | O95954 |
| ERAB | Q99714 |
| HSP 70 | P0DMV8 |
| XPNPEP1 | Q9NQW7 |
| PHI | P06744 |
| sE-Selectin | P16581 |
| AK1A1 | P14550 |
| HTRA2 | O43464 |
| YES | P07947 |
| ApoM | O95445 |
| AMPM2 | P50579 |
| C1-Esterase Inhibitor | P05155 |
| HSP70 protein 8 | P11142 |
| PIGR | P01833 |
| CD30 Ligand | P32971 |
| IGFBP-5 | P24593 |
| PLXB2 | 015031 |
| IL-19 | Q9UHD0 |
| sCD163 | Q86VB7 |
| MDHC | P40925 |
| PLXC1 | O60486 |
| IL-1 sRII | P27930 |
| HEMK2 | Q9Y5N5 |
| Testican-1 | Q08629 |
| NSE | P09104 |
| Cathepsin D | P07339 |

**Table 2F. Protein Markers for Hepatic Ballooning, Filtered (not according to the invention)**

| **Marker Name** | **UniProt** |
|---|---|
| ERAB | Q99714 |
| PHI | P06744 |
| XPNPEP1 | Q9NQW7 |
| Cathepsin D | P07339 |
| FTCD | O95954 |
| HSP 70 | P0DMV8 |
| AK1A1 | P14550 |
| sCD163 | Q86VB7 |
| HEMK2 | Q9Y5N5 |
| ApoM | O95445 |
| sE-Selectin | P16581 |
| Testican-1 | Q08629 |
| HSP70 protein 8 | P11142 |
| Cytochrome c | P99999 |
| SHP-2 | Q06124 |
| CD30 Ligand | P32971 |
| C1-Esterase Inhibitor | P05155 |
| YES | P07947 |
| PIGR | P01833 |
| PLXC1 | O60486 |
| IL-1 sRI | P14778 |
| CAMK2D | Q13557 |
| FABPL | P07148 |
| HSP 60 | P10809 |
| Gelsolin | P06396 |
| CD48 | P09326 |
| PSA | P07288 |
| IGFBP-5 | P24593 |
| MMP-7 | P09237 |
| IL-19 | Q9UHD0 |
| AMPM2 | P50579 |
| Nectin-like protein 2 | Q9BY67 |
| Glutamate carboxypeptidase | Q96KP4 |
| CATZ | Q9UBR2 |
| ENPP7 | Q6UWV6 |
| PLXB2 | 015031 |
| IL-18 BPa | O95998 |
| STAT1 | P42224 |
| FSTL3 | O95633 |
| IL-18 Ra | Q13478 |
| IL-1 R AcP | Q9NPH3 |

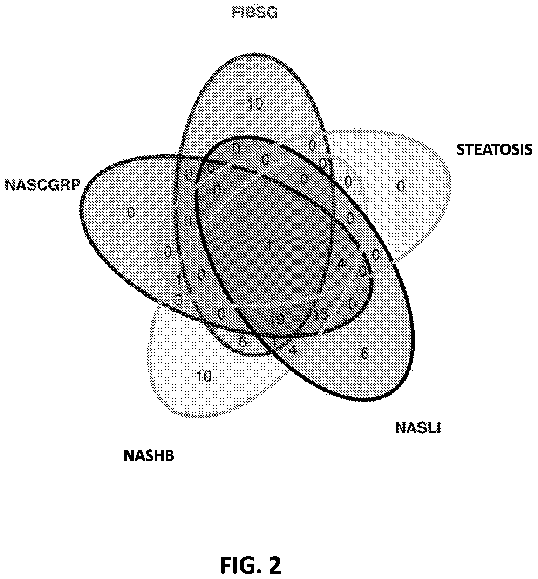

**FIG. 2** shows common protein markers are present between different diagnostic variables (FIBSG: CRN fibrosis stages; STEATOSI: steatosis; NASLI: NAS Lobular Inflammation; NASHB: NAS Hepatic Ballooning; NASCGRP: NAS score). Certain overlaps are summarized in Table A below:

**Table A. Common Protein Markers between Groups**

| **Group** | **Markers** |
|---|---|
| Unique to CRN fibrosis (10) | 6Ckine, BSSP4, IL-8, LIF sR, LTBP4, MIC-1, SAP, SEM6B, SLAF7, Spondin-1 |
| Unique to HB (10) | CATZ, FABPL, Gelsolin, Glutamate carboxypeptidase, HSP 60, IL-1 R AcP, IL-18 Ra Nectin-like protein 2, PSA, STAT1 |
| Unique to L (6) | HTRA2, IL-1 sRII, MDHC, NAGK, NSE, PIK3CA/PIK3R1 |
| Common to all 5 responsive variables (1) | HSP 70 |
| Common to CRN fibrosis/HB (6) | CD48, FSTL3, IL-1 sRI, IL-18 BPa, MMP-7, SHP-2 |
| Common to CRN fibrosis/HS/LI (1) | IGFBP-7 |
| Common to CRN fibrosis/NAS/HB/LI (10) | C7, CD30 Ligand, Collectin Kidney 1, ERAB, IL-19, NADPH-P450 Oxidoreductase, sCD163, sE-Selectin TSP2, XPNPEP1 |
| Common to NAS/HB/LI/Steatosis (4) | Aminoacylase-1, HSP 90a/b, HSP 90b, Integrin a1b |
| Common to NAS/HB (3) | Cytochrome c, ENPP7, M-CSF R |
| Common to HB/LI(4) | dopa decarboxylase, IGFBP-5, PLXB2, PLXC |
| Common to NAS/HB/Steatosis (1) | CAMK2D |
| Common to NAS/HB/LI (13) | AK1A1, AMPM2,ApoM, C1-Esterase Inhibitor, Cathepsin D, FTCD, HEMK2, HSP70 protein 8, KYNU, PHI, PmGR, Testican-1, YES |

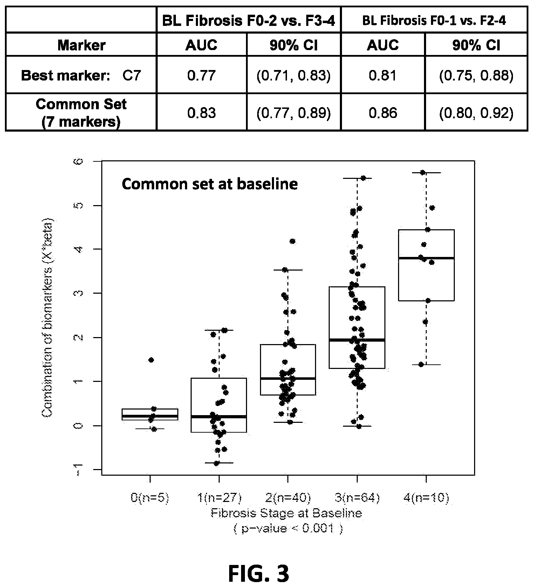

Multivariate analysis further identified a panel of 7 protein markers (C7, CL-K1, IGFBP7, Spondin 1, IL-5Ra (UniProt: Q01344), MMP-7 and TSP2) that possess good diagnostic value to classify NASH subjects with severe fibrosis (F0-1 vs F3-4; AUROC: 0.83; **FIG. 3**). Changes in circulating levels of the biomarkers were generally reflected in the expression of their corresponding RNAs by RNAseq of formalin-fixed paraffin-embedded (FFPE) sections of liver.

Longitudinal changes of several protein markers (KYNU, Integrin a1b1, CL-K1, C7, ACY1 and TSP2) that are associated with fibrosis stage or NAS score at baseline also significantly correlate with improvement in one or more NASH clinical features (fibrosis, steatosis and lobular inflammation). The detailed listing of protein markers for monitoring clinical improvement features are provided in Tables 3A-D below.

### Tables 3A-D: Protein markers for monitoring clinical improvements

**Table 3A. Protein Biomarkers for Monitoring Steatosis Improvement (not according to the invention)**

| **Marker Name** | **UniProt** | **Median percent CHG at W24 in Non-improver** | **Median percent CHG at W24 in improver** | **p value** |
|---|---|---|---|---|
| KYNU | Q16719 | -6.785 | -30.97 | 6.57E-05 |
| Nectin-like protein 2 | Q9BY67 | 7.925 | -7.93 | 0.000107 |
| CD30 Ligand | P32971 | 0.81 | -9.18 | 0.000215 |
| YKL-40 | P36222 | 13.28 | -14.29 | 0.000368 |
| TSP2 | P35442 | 21.215 | -25.59 | 0.000493 |
| PCSK7 | Q16549 | 7.995 | -3.57 | 0.000522 |
| IR | P06213 | 0.875 | -13.12 | 0.000576 |
| AK1A1 | P14550 | -8.95 | -28.48 | 0.000586 |
| FLRT3 | Q9NZU0 | 3.09 | -10.72 | 0.000586 |
| TIMD3 | Q8TDQ0 | 4.615 | -8.75 | 0.00062 |
| SAP | P02743 | -1.14 | 5.44 | 0.000695 |
| YES | P07947 | -0.1 | -11.71 | 0.000706 |
| Integrin a1b1 | P56199, P05556 | -5.025 | -41.26 | 0.000724 |
| CNTN2 | Q02246 | 3.91 | -6.28 | 0.000821 |
| sTie-2 | Q02763 | -0.925 | -8.34 | 0.000821 |
| Lumican | P51884 | 4.69 | -6.81 | 0.000821 |
| HSP 70 | P0DMV8 | -0.875 | -27.05 | 0.000868 |

**Table 3B. Protein Biomarkers for Monitoring Lobular Inflammation Improvement (not according to the invention)**

| **Marker Name** | **UniProt** | **Median percent CHG at W24 in Non-improver** | **Median percent CHG at W24 in improver** | **p value** |
|---|---|---|---|---|
| M-CSF R | P07333 | 5.72 | -12.625 | 4.58E-05 |
| ACE2 | Q9BYF1 | -2.05 | 2.605 | 0.000138 |
| FCG3B | 075015 | -1.44 | -14.29 | 0.000191 |
| IL24 | Q13007 | 2.03 | -3.985 | 0.00038 |
| ERAB | Q99714 | 2.01 | -26.665 | 0.000429 |
| Keratin 18 | P05783 | -0.96 | 4.1 | 0.000515 |
| Aminoacylase-1 | Q03154 | -11.97 | -37.08 | 0.000727 |
| PHI | P06744 | -5.37 | -30.06 | 0.000862 |
| C4 | P0C0L4, P0C0L5 | -2.53 | 7.51 | 0.001019 |
| HSP 90a/b | P07900 | -4.98 | -21.04 | 0.001202 |
| | P08238 | | | |
| CHST2 | Q9Y4C5 | 1.67 | -5.34 | 0.001291 |
| Integrin a1b1 | P56199, P05556 | -5.31 | -34.885 | 0.00195 |
| PIK3CA/PIK3R1 | P42336 | -2.3 | -13.095 | 0.001996 |
| | P27986 | | | |
| Cathepsin D | P07339 | 4.99 | -14.74 | 0.002711 |
| NADPH-P450 Oxidoreductase | P16435 | -8.01 | -37.395 | 0.002777 |
| CBG | P08185 | -2.61 | 1.495 | 0.003557 |
| MDM2 | Q00987 | 1.43 | -3.26 | 0.004668 |
| C7 | P10643 | 5.79 | -7.535 | 0.004745 |
| CD5L | O43866 | 6.76 | -6.625 | 0.00581 |
| IL-8 | P10145 | -4.72 | -15.795 | 0.005992 |
| LYVE1 | Q9Y5Y7 | 4.61 | -4.21 | 0.00661 |
| Eotaxin | P51671 | 4.34 | 15.185 | 0.007197 |
| Albumin | P02768 | -0.65 | 13.88 | 0.007351 |
| Histone H2A.z | P0C0S5 | 10.31 | -8.545 | 0.007519 |
| KIF23 | Q02241 | -2.64 | 2.93 | 0.007519 |
| ALT | P24298 | -0.07 | -9.27 | 0.008514 |
| EPO-R | P19235 | -1.13 | 2.2 | 0.008591 |
| STX1a | Q16623 | 3.03 | -2.59 | 0.00906 |
| MED-1 | Q15648 | -2.61 | 11.545 | 0.009378 |

**Table 3C. Protein Biomarkers for Monitoring Hepatic Ballooning Improvement (not according to the invention)**

| **Marker Name** | **UniProt** | **Median percent CHG at W24 in Non-improver** | **Median percent CHG at W24 in improver** | **p value** |
|---|---|---|---|---|
| Protein C | P04070 | -0.09 | 9.69 | 0.000589 |
| LG3BP | Q08380 | 7.44 | -7.56 | 0.006607 |
| Cathepsin D | P07339 | 4.95 | -15.96 | 0.01054 |
| Coagulation Factor Xa | P00742 | 0.825 | 6.52 | 0.010541 |
| Thyroxine-Binding Globulin | P05543 | -1.5 | -7.58 | 0.014594 |
| XEDAR | Q9HAV5 | -0.875 | 1.66 | 0.014887 |
| CBG | P08185 | -2.655 | 1.44 | 0.015188 |
| HGF | P14210 | -4.445 | -9.9 | 0.015802 |
| LTBP4 | Q8N2S1 | 2.525 | -5.93 | 0.017776 |
| Eotaxin-2 | 000175 | -0.04 | 4.07 | 0.018122 |
| B7-H2 | 075144 | -5.27 | -12.52 | 0.01921 |
| JAG1 | P78504 | 2.6 | 0.16 | 0.019963 |
| STAT3 | P40763 | -4.58 | -8.59 | 0.020743 |
| VEGF sR2 | P35968 | -1.275 | 4.03 | 0.020744 |
| Laminin | P25391, P07942, P11047 | 2.15 | -8.68 | 0.021551 |
| Topoisomerase I | P11387 | 6.47 | -13.48 | 0.021551 |
| IGFBP-5 | P24593 | -0.695 | 6.44 | 0.023245 |
| Heparin cofactor II | P05546 | -1.345 | 3.88 | 0.024135 |
| Osteopontin | P10451 | -1.54 | 1.79 | 0.024585 |
| BAFF | Q9Y275 | 2.16 | -7.94 | 0.026002 |
| IL-13 Ra1 | P78552 | -0.565 | 5.91 | 0.026002 |
| PH | P01298 | 6.14 | -3.88 | 0.026004 |
| EphA1 | P21709 | 1.83 | 8.65 | 0.026984 |
| HINT1 | P49773 | -10.655 | -30.56 | 0.026984 |
| kallikrein 12 | Q9UKR0 | -1.51 | 3.44 | 0.026984 |
| M2-PK | P14618 | -3.615 | -22.55 | 0.026984 |
| Lymphotoxin b R | P36941 | -0.575 | 3.35 | 0.027996 |
| C3 | P01024 | -3.82 | -24.36 | 0.029041 |
| pTEN | P60484 | -0.705 | -5.01 | 0.029041 |
| Fucosyltransferase 3 | P21217 | 3.205 | -1.89 | 0.031232 |
| Myeloperoxidase | P05164 | -9.61 | -17.96 | 0.031232 |
| Tropomyosin 2 | P07951 | -0.98 | 4.09 | 0.032377 |
| ARMEL | Q49AH0 | 1.91 | 10.94 | 0.032379 |
| PKB beta | P31751 | -0.495 | -7.77 | 0.032379 |
| I-309 | P22362 | -2.48 | 7.66 | 0.03356 |
| GDF2 | Q9UK05 | -10.91 | -21.82 | 0.033562 |
| TrkA | P04629 | -2.235 | 5.74 | 0.033562 |
| PLXB2 | 015031 | 3.24 | -3.7 | 0.03478 |
| Proteinase-3 | P24158 | -14.585 | -26.62 | 0.034782 |
| S100A7 | P31151 | 1.06 | -1.99 | 0.03604 |
| ARGI1 | P05089 | -6.98 | -19.27 | 0.037336 |
| sCD163 | Q86VB7 | 2.88 | -4.88 | 0.037995 |
| Adrenomedullin | P35318 | 1.22 | -5.44 | 0.038672 |
| MAPK14 | Q16539 | -0.58 | -5.45 | 0.040046 |
| TMA | P07202 | -0.93 | 2.55 | 0.040048 |
| Epo | P01588 | 18.195 | 1.25 | 0.041464 |
| annexin I | P04083 | 2.89 | -12.32 | 0.042189 |
| BPI | P17213 | -20.905 | -39.76 | 0.044429 |
| Collectin Kidney 1 | Q9BWP8 | -1.64 | -13.64 | 0.044429 |
| IL-17F | Q96PD4 | -1.62 | 4.93 | 0.044429 |
| JAM-B | P57087 | -0.86 | 6.48 | 0.044429 |
| Integrin aVb5 | P06756, P18084 | 1.765 | -5.42 | 0.045976 |
| MP2K2 | P36507 | 1.985 | 10.3 | 0.045976 |
| SLIK1 | Q96PX8 | -0.975 | 5.51 | 0.045976 |
| SP-D | P35247 | -7.39 | -30.86 | 0.045976 |
| CD63 | P08962 | -1.285 | 4.6 | 0.048379 |
| KEAP1 | Q14145 | -0.79 | 5.74 | 0.049202 |
| calgranulin B | P06702 | -11.515 | -28.5 | 0.049204 |
| PSD7 | P51665 | 1.45 | -0.75 | 0.049204 |
| RNase H1 | O60930 | 0.585 | -4.06 | 0.049204 |
| GNS | P15586 | 3.03 | -6.64 | 0.049207 |

**Table 3D. Protein Biomarkers for Monitoring CRN Fibrosis Stage Improvement**

| **Marker Name** | **UniProt** | **Median percent CHG at W24 in Non-improver** | **Median percent CHG at W24 in improver** | **p value** |
|---|---|---|---|---|
| GITR | Q9Y5U5 | -1.84 | -7.67 | 0.00178 |
| Cathepsin H | P09668 | -3.02 | -13.86 | 0.0038 |
| DcR3 | O95407 | -2.01 | 1.1 | 0.005199 |
| LAG-1 | Q8NHW4 | -4.56 | -11.84 | 0.00619 |
| PDXK | O00764 | -2.82 | -13.7 | 0.00766 |
| RNase H1 | O60930 | -2.6 | 2.2 | 0.007865 |
| GP1BA | P07359 | -4.35 | 1.52 | 0.009824 |
| IL-1a | P01583 | -0.12 | -4.67 | 0.011627 |
| GIB | P04054 | 2.12 | -12.9 | 0.012548 |
| carbonic anhydrase II | P00918 | -2 | 4.59 | 0.01301 |
| Caspase-2 | P42575 | -2.27 | 2.84 | 0.015708 |
| Cystatin-S | P01036 | -1.78 | 3.35 | 0.016421 |
| Troponin I, skeletal, fast twitch | P48788 | 6.48 | -1.77 | 0.018398 |
| IF4A3 | P38919 | -1.61 | 2.58 | 0.02063 |
| PSMA | Q04609 | 1.42 | -6.53 | 0.021352 |
| Dtk | Q06418 | 2.32 | -1.06 | 0.022546 |
| LY9 | Q9HBG7 | 3.6 | -6.33 | 0.022975 |
| MK13 | 015264 | -4.33 | 0.54 | 0.024616 |
| KI3L2 | P43630 | -1.21 | 1.92 | 0.024702 |
| TrkC | Q16288 | 3.16 | -4.72 | 0.026387 |
| PCI | P05154 | 12.19 | 1.79 | 0.026539 |
| TNFSF15 | 095150 | 2.31 | -2.49 | 0.026539 |
| Coagulation Factor IX | P00740 | 6.7 | 0.35 | 0.0275 |
| MMP-3 | P08254 | 12.69 | 2.97 | 0.027786 |
| INGR2 | P38484 | -1 | 2.76 | 0.028756 |
| C3a | P01024 | -19.78 | 15.9 | 0.029511 |
| LRP8 | Q14114 | 1.37 | -2.81 | 0.029511 |
| 17-beta-HSD 1 | P14061 | -4.14 | 2.16 | 0.029753 |
| MP2K3 | P46734 | -3.46 | -0.97 | 0.030562 |
| CD70 | P32970 | -1.88 | 3.16 | 0.031303 |
| CPNE1 | Q99829 | -4.36 | 2.87 | 0.031645 |
| NEUREGULIN-1 | Q02297 | -0.69 | 6.86 | 0.031645 |
| PCSK9 | Q8NBP7 | -2.85 | 8.12 | 0.031645 |
| pTEN | P60484 | -4.94 | 2.32 | 0.031645 |
| K-ras | P01116 | 0.27 | -3.79 | 0.033907 |
| ATS15 | Q8TE58 | -0.15 | 4.2 | 0.036304 |
| Ephrin-A5 | P52803 | 5.39 | -0.13 | 0.036304 |
| Endoglin | P17813 | 1.62 | -3.86 | 0.037555 |
| AFP | P02771 | -0.39 | 1.07 | 0.037603 |
| FTCD | O95954 | -6.71 | -42.29 | 0.037603 |
| Granzyme B | P10144 | -1.2 | 2.74 | 0.038842 |
| Activin RIB | P36896 | -2.91 | 0.64 | 0.038856 |
| TGF-b3 | P10600 | -0.94 | 2.97 | 0.040146 |
| DSC3 | Q14574 | 1.23 | 6.37 | 0.040165 |
| WFKN2 | Q8TEU8 | 0.47 | -2.98 | 0.042148 |
| c-Myc | P01106 | -2.91 | 0.18 | 0.043526 |
| b-Endorphin | P01189 | -0.56 | 2.33 | 0.043529 |
| MICA | Q29983 | 1.73 | -5.58 | 0.044362 |
| ART | O00253 | 9.05 | -1.82 | 0.04584 |
| CD30 Ligand | P32971 | -0.44 | -4.89 | 0.047903 |
| GV | P39877 | 0.22 | 3.21 | 0.048664 |
| CLC7A | Q9BXN2 | -3.19 | 3.23 | 0.048918 |
| IL-17D | Q8TAD2 | 0.85 | -1.33 | 0.048918 |
| KYNU | Q16719 | -8.6 | -26.22 | 0.048918 |
| SLAF5 | Q9UIB8 | -4.04 | -0.59 | 0.048918 |
| SSRP1 | Q08945 | -1.51 | -0.48 | 0.049438 |

The markers were further manually filtered with their activities and other information and the filtered lists are presented in Tables 4A-D below.

### Tables 4A-D: Protein markers for monitoring clinical improvements, filtered

**Table 4A. Protein Biomarkers for Monitoring Steatosis Improvement, Filtered (not according to the invention)**

| **Marker Name** | **UniProt** |
|---|---|
| Nectin-like protein 2 | Q9BY67 |
| CD30 Ligand | P32971 |
| YKL-40 | P36222 |
| TSP2 | P35442 |
| PCSK7 | Q16549 |
| IR | P06213 |
| AK1A1 | P14550 |
| FLRT3 | Q9NZU0 |
| TIMD3 | Q8TDQ0 |
| SAP | P02743 |
| YES | P07947 |
| CNTN2 | Q02246 |
| sTie-2 | Q02763 |
| Lumican | P51884 |
| HSP 70 | P0DMV8 |

**Table 4B. Protein Biomarkers for Monitoring Lobular Inflammation Improvement, Filtered (not according to the invention)**

| **Marker Name** | **UniProt** |
|---|---|
| ACE2 | Q9BYF1 |
| IL24 | Q13007 |
| ERAB | Q99714 |
| Keratin 18 | P05783 |
| PHI | P06744 |
| C4 | P0C0L4, P0C0L5 |
| CHST2 | Q9Y4C5 |
| Cathepsin D | P07339 |
| CBG | P08185 |
| MDM2 | Q00987 |
| CD5L | O43866 |
| IL-8 | P10145 |
| LYVE1 | Q9Y5Y7 |
| Eotaxin | P51671 |
| Albumin | P02768 |
| Histone H2A.z | P0C0S5 |
| KIF23 | Q02241 |
| ALT | P24298 |
| EPO-R | P19235 |
| STX1a | Q16623 |
| MED-1 | Q15648 |

**Table 4C. Protein Biomarkers for Monitoring Hepatic Ballooning Improvement, Filtered (not according to the invention)**

| **Marker Name** | **UniProt** |
|---|---|
| Protein C | P04070 |
| LG3BP | Q08380 |
| Cathepsin D | P07339 |
| Coagulation Factor Xa | P00742 |
| Thyroxine-Binding Globulin | P05543 |
| XEDAR | Q9HAV5 |
| CBG | P08185 |
| HGF | P14210 |
| LTBP4 | Q8N2S1 |
| Eotaxin-2 | 000175 |
| B7-H2 | 075144 |
| JAG1 | P78504 |
| STAT3 | P40763 |
| VEGF sR2 | P35968 |
| Laminin | P25391, P07942, P11047 |
| Topoisomerase I | P11387 |
| IGFBP-5 | P24593 |
| Heparin cofactor II | P05546 |
| Osteopontin | P10451 |
| BAFF | Q9Y275 |
| IL-13 Ra1 | P78552 |
| PH | P01298 |
| EphA1 | P21709 |
| HINT1 | P49773 |
| kallikrein 12 | Q9UKR0 |
| M2-PK | P14618 |
| Lymphotoxin b R | P36941 |
| C3 | P01024 |
| pTEN | P60484 |
| Fucosyltransferase 3 | P21217 |
| Myeloperoxidase | P05164 |
| Tropomyosin 2 | P07951 |
| ARMEL | Q49AH0 |
| PKB beta | P31751 |
| I-309 | P22362 |
| GDF2 | Q9UK05 |
| TrkA | P04629 |
| PLXB2 | 015031 |
| Proteinase-3 | P24158 |
| S100A7 | P31151 |
| ARGI1 | P05089 |
| sCD163 | Q86VB7 |
| Adrenomedullin | P35318 |
| MAPK14 | Q16539 |
| TMA | P07202 |
| Epo | P01588 |
| annexin I | P04083 |
| BPI | P17213 |
| IL-17F | Q96PD4 |
| JAM-B | P57087 |
| Integrin aVb5 | P06756, P18084 |
| MP2K2 | P36507 |
| SLIK1 | Q96PX8 |
| SP-D | P35247 |
| CD63 | P08962 |
| KEAP1 | Q14145 |
| calgranulin B | P06702 |
| PSD7 | P51665 |
| RNase H1 | O60930 |
| GNS | P15586 |

**Table 4D. Protein Biomarkers for Monitoring CRN Fibrosis Stage Improvement, Filtered**

| **Marker Name** | **UniProt** |
|---|---|
| GITR | Q9Y5U5 |
| Cathepsin H | P09668 |
| DcR3 | O95407 |
| LAG-1 | Q8NHW4 |
| PDXK | O00764 |
| RNase H1 | O60930 |
| GP1BA | P07359 |
| IL-1a | P01583 |
| GIB | P04054 |
| carbonic anhydrase II | P00918 |
| Caspase-2 | P42575 |
| Cystatin-S | P01036 |
| Troponin I, skeletal, fast twitch | P48788 |
| IF4A3 | P38919 |
| PSMA | Q04609 |
| Dtk | Q06418 |
| LY9 | Q9HBG7 |
| MK13 | 015264 |
| KI3L2 | P43630 |
| TrkC | Q16288 |
| PCI | P05154 |
| TNFSF15 | 095150 |
| Coagulation Factor IX | P00740 |
| MMP-3 | P08254 |
| INGR2 | P38484 |
| C3a | P01024 |
| LRP8 | Q14114 |
| 17-beta-HSD 1 | P14061 |
| MP2K3 | P46734 |
| CD70 | P32970 |
| CPNE1 | Q99829 |
| NEUREGULIN-1 | Q02297 |
| PCSK9 | Q8NBP7 |
| pTEN | P60484 |
| K-ras | P01116 |
| ATS15 | Q8TE58 |
| Ephrin-A5 | P52803 |
| Endoglin | P17813 |
| AFP | P02771 |
| FTCD | O95954 |
| Granzyme B | P10144 |
| Activin RIB | P36896 |
| TGF-b3 | P10600 |
| DSC3 | Q14574 |
| WFKN2 | Q8TEU8 |
| c-Myc | P01106 |
| b-Endorphin | P01189 |
| MICA | Q29983 |
| ART | O00253 |
| CD30 Ligand | P32971 |
| GV | P39877 |
| CLC7A | Q9BXN2 |
| IL-17D | Q8TAD2 |
| SLAF5 | Q9UIB8 |
| SSRP1 | Q08945 |

Multivariate analysis for the monitoring markers also identifies a few groups of markers, when used collectively, possess better monitoring capabilities. These multivariate marker groups are listed in Table B below.

**Table B. Multivariate Protein Markers for Treatment Monitoring**

| **Endpoint** | **Protein Markers** | | **FIG.** |
|---|---|---|---|
| CRN Fibrosis stage | **Marker** | **(UniProt)** | **FIG. 4** |
| | pTEN | (P60484) | |
| | CD70 | (P32970) | |
| | Caspase-2 | (P42575) | |
| | Cathepsin H | (P09668) | |
| | LAG-1 | (Q8NHW4) | |
| | PDXK | (O00764) | |
| | GITR | (Q9Y5U5) | |
| Steatosis | **Marker** | **(UniProt)** | **FIG. 5** |
| | Integrin a1b1 | (P56199, P05556) | |
| | Nectin-like protein 2 | (Q9BY67) | |
| | PDGF Rb | (P09619) | |
| | LRP8 | (Q14114) | |
| | CD30 Ligand | (P32971) | |
| | Lumican | (P51884) | |
| | SAP | (P02743) | |
| | YKL-40 | (P36222) | |
| | sTie-2 | (Q02763) | |
| | HSP 90a/b | (P07900 P08238) | |
| | TSP2 | (P35442) | |
| | YES | (P07947) | |
| Lobular Inflammation | **Marker** | **(UniProt)** | **FIG. 6** |
| | HSP 90a/b | (P07900 P08238) | |
| | Aminoacylase-1 | (Q03154) | |
| | FCG3B | (075015) | |
| | M-CSF R | (P07333) | |
| | Keratin 18 | (P05783) | |
| Hepatic Ballooning | **Marker** | **(UniProt)** | **FIG. 7** |
| | Fibronectin | (P02751) | |
| | Thyroxine-Binding Globulin | (P05543) | |
| | FGF23 | (Q9GZV9) | |
| | LG3BP | (Q08380) | |
| | Heparin cofactor | II (P05546) | |
| | Protein C | (P04070) | |
| | STAT3 | (P40763) | |

This example identifies new protein biomarker candidates for staging fibrosis, steatosis, lobular inflammation, and hepatic ballooning in NASH subjects are identified using SOMAscan. Additionally, in F2-3 NASH subjects treated with selonsertib, markers that show treatment response monitoring characteristics are also identified.

### Example 2. Serum bile acid levels are reciprocally regulated with C4 levels across the spectrum of disease severity in patients with nonalcoholic steatohepatitis (NASH) (not according to the invention)

Serum bile acid (BA) profiles are altered in patients with NASH. Data describing associations between BA composition and fibrosis stage are limited. This example was performed to: 1) characterize circulating BAs and its intermediate, 7α-hydroxy-4-cholesten-3-one (C4), in patients across the spectrum of NASH and heathy controls; and 2) determine associations between serum BAs and fibrosis stage in NASH.

### Methods:

Fasting serum levels of 15 BAs were quantified in healthy controls (n=118), NASH F2/3 (n=72), and NASH cirrhosis (n=29) by LC-MS/MS (Agilent 1290/Sciex, Metabolon). Serum from clinical studies of healthy controls in PK studies of Compound A (a selective, non-steroidal agonist of the Farnesoid X receptor), simtuzumab in F2/3 fibrosis, and cirrhosis was used. Analysis of serum BA from cirrhosis patients occurred prior and after clinical decompensation. Serum C4 levels were measured to reflect hepatic bile acid biosynthesis. One-way ANOVA was used to assess differences in BA and C4 levels between groups. Data presented as median (IQR).

### Results:

Fasting total serum BA levels in healthy controls were 877ng/mL (582, 1298) and increased in a step-wise fashion with progressive fibrosis: F2, 1483 (937, 2356); F3, 1825 (1226, 2848); F4, 12,161 (5614, 22,112). The ~14-fold increase in total serum BA in NASH cirrhosis consisted primarily of an increase in conjugated BAs 11,518 (4737, 17,572) compared with F2 NASH, 1141 (431, 1714). Primary conjugated bile acids [glycocholate (GCA), taurocholate (TCA), glycodeoxycholate (GCDCA), and taurodeoxycholate (TCDCA)] were the major primary BA species elevated in F4 vs F2 NASH. GCA and TCA were ~15 and 35-fold higher, respectively, in F4 compared to F2 NASH.

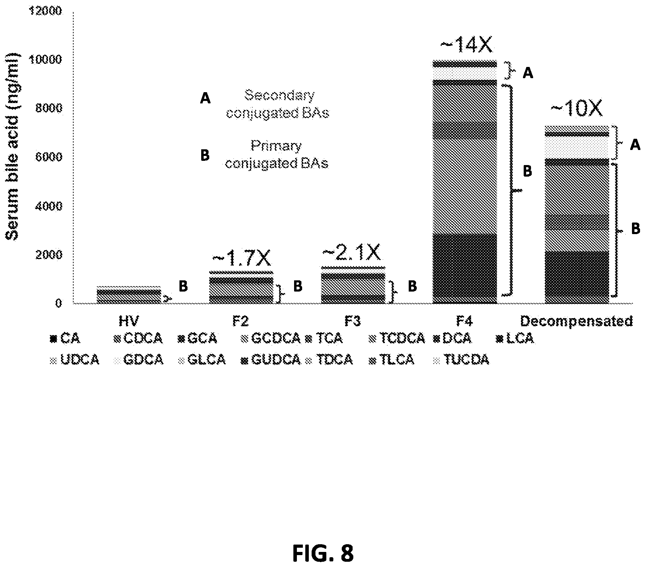

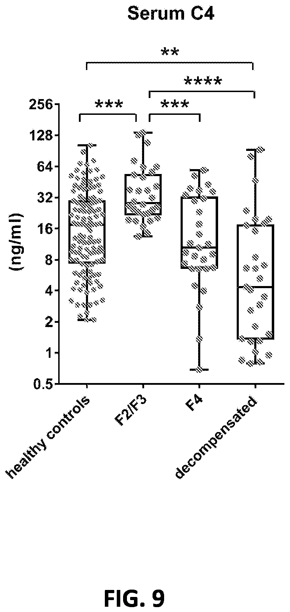

Clinical decompensation of NASH cirrhosis subjects led to decreased BA levels, but at levels still ~10-fold higher than controls (**FIG. 8**). In contrast, BA synthesis as reflected in serum C4 levels declined with the development of cirrhosis and further decreased with clinical decompensation (**FIG. 9**). C4 levels in F2/F3 patients, 28.4 ng/mL (21.5, 54.1), were significantly higher than controls, 17.4 (7.3, 30.0). Development of cirrhosis resulted in C4 levels that were 63% lower than F2/F3 patients, 10.5 (6.6, 32.8) and decreased further with clinical decompensation, 4.3 (1.3, 17.6).

An increase in overall BAs and conjugated primary BAs occurs in F2/F3 NASH compared to healthy controls and is accompanied by elevated C4 levels. In NASH cirrhosis, pronounced elevation in BAs occurs despite lower C4 levels than those found in F2/F3 NASH. These results indicate that the mechanisms responsible for BA homeostasis are lost in cirrhosis, and become further dysregulated with clinical decompensation.

### Example 3. Extracellular Proteome- RNAseq Analysis for Identifying Non- Invasive Nash-Fibrosis Biomarker

This example used a combination of NASH biopsy-derived transcriptomics analysis and predictive bioinformatics algorithms to identify 100 transcripts that could produce secreted/leaked proteins (so called NASH secretome). These transcripts exhibited fibrosis stage dependent expression profiles and are relevant to NASH biology. Individual or combined transcripts are good discriminators (AUROC > 0.86) for classifying NASH subjects with cirrhosis (F4) or severe fibrosis (F3/F4).

ELISA assays were selected and qualified for the top 30 candidates. 11 proteins (YKL-40, FAP, ITGB6, EMILIN1, FNDC1, IGDCC4, MASP2, SCF, LTBP2, ADAMTS12 and MCM2) exhibited significant association with CRN fibrosis in F0-F4 samples. FNDC1 and MCM2 (AUROC = 0.80 and 0.76, respectively) are good discriminators of severe fibrosis (F ≥ 3). Longitudinal changes of the 11 markers are not associated with fibrosis improvement or worsening. Changes in YKL-40, FAP, and SCF (AUROC = 0.70, 0.67 and 0.72, respectively) are fair monitoring markers of steatosis improvement. Baseline levels of FAP, SCF, and LTBP2 are fair prognostic markers (AUROC = 0.78, 0.71 and 0.70, respectively) of fibrosis improvement in F4 subjects.

In Example 1, assays for selective protein targets (LOXL2, Lumican, TGFBI, CK- 18s, Pro-C3) as well as high-content proteomic platform (SOMAscan) were employed as part of the comprehensive proteomic approaches to identify and evaluate novel protein markers for NASH. Even though about 1350 proteins were covered by these two approaches, there are still many potential circulating proteins that are not included. As a complementary proteomic approach, NASH biopsy-derived transcriptomics analysis was combined with predictive bioinformatics algorithms to identify additional secreted/leaked proteins (so called NASH secretome) from liver for further exploration.

Proteins in circulation may come from 1) "Classic secretory" via exocytosis, 2) "Non-classic secretory" through translocation, lysosomal secretion or exosome, or 3) tissue leakage due to cell death or damage.

In order to predict changes in potential circulating proteins, transcriptomic information from tissues of interest to predict potential secreted or leaked protein. This example developed bioinformatics algorithms to predict genes to 1) demonstrate fibrosis stage dependent differential expression in NASH subjects and 2) encode for secreted proteins. These NASH secretome will represent potential tissue-selective and disease severity dependent candidates as circulating protein biomarkers. After these candidates were identified, protein quantification data were either generated using ELISA or derived from SOMAscan if available.

### Samples

Procured FFPE liver samples listed in Table 5 were used for RNA extraction and RNAseq

**Table 5. Procured Samples for RNAseq**

| | **Healthy** | **F1** | **F2** | **F3** | **F4** |
|---|---|---|---|---|---|
| Phase I: pilot (completed) | 6* | 4 | 14 | 11 | 1 |
| Phase 2: second dataset (initiated, data expected in May) | 21 | 46 | 27 | 5 | 10 |
| Total | 27 | 50 | 41 | 16 | 11 |

### ELISA Testing for Selected Candidates

Procured serum samples were used for initial ELISA screening experiments to identify candidates for further testing using clinical study samples.

### Bioinformatics Methods for Candidate Selection

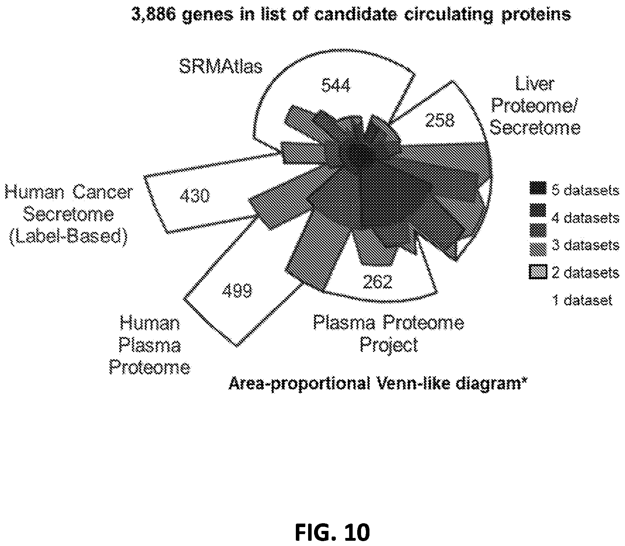

Genes with experimental/MS evidence of secretion were identified in public databases/datasets. Collectively, about 3886 potential secreted or leaked proteins were identified and overlapped or unique proteins from each dataset are illustrated in **FIG. 10****.**

A candidate list of non-invasive, circulating biomarkers for NASH progression using RNA-seq data and public databases/datasets was then compiled with a bioinformatic workflow.

### NASH Secretome Experimental Workflow

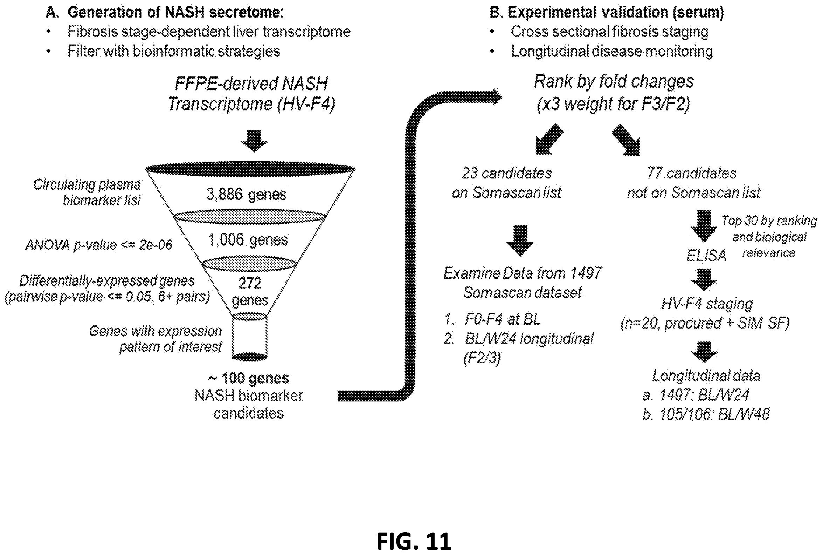

About 100 genes were selected after the filtering procedures mentioned above. These potential fibrosis stage-dependent, liver selective secreted proteins were then going through experimental validation either by SOMAscan (depends on availability) or ELISA testing (**FIG. 11**).

### Objectives

As a complementary proteomic approach, bioinformatics algorithms were employed to predict potential hepatic secreted/leaked proteins from NASH subjects. Circulating levels of promising candidates are measured using ELISA assays or SOMAscan (depends on availability) to examine association of: levels of these protein markers with fibrosis stages at BL; longitudinal changes of these markers with improvement/worsening of liver fibrosis in NASH subjects.

Evaluate the *performance* (i.e., AUROC) of markers from secretome panel for *diagnosing* advanced fibrosis (F3-4 vs. F0-2) and cirrhosis (F4 vs. F0-3) in combined datasets.

Evaluate the *performance* of markers secretome panel for *monitoring* CRN fibrosis stage change.

Evaluate the *performance* of markers secretome panel for *prognosing* CRN fibrosis stage change (improvement and worsening).

Evaluate the *performance* of markers secretome panel for *monitoring* improvement in NAS components (hepatic ballooning, lobular inflammation and steatosis).

Evaluate the *performance* of markers secretome panel for *diagnosing* NASH vs. non-NASH in combined dataset across studies SEL1497, SIM 0105 and 0106, where non- NASH is evaluated using the following four definitions: 1) 0 in any NAS subscore (lobular inflammation, hepatic ballooning and steatosis), 2) no to mild inflammation (NASLI ≤ 1), 3) no ballooning (NASHB = 0), 4) no active NASH (NASLI ≤ 1 and NASHB = 0).

### Statistical Methods

For AUROC calculation for binary endpoint, the method of repeated cross-validation was performed. Specifically, the data is randomly divided into 5-folds, using 4 of the folds for the modeling (i.e. logistic regression) and predicting on the left-out fold, and performing the modeling/predicting process for each of the 5-folds until the predictions are obtained for all 5 folds (i.e. whole dataset). AUROC performance metric is obtained. The cross-validation procedure is then repeated 100 times with a different randomly-divided 5 folds each time, and the mean and 95% CI for the AUROC are provided across the 100 repeats. In the modeling, logistic regression was used to model the binary endpoint (e.g. baseline F4 vs. F0-3) with the biomarker(s) as covariate.

Additional analyses for baseline CRN fibrosis stage are provided. In particular, boxplots of baseline levels of specific conventional tests by baseline CRN fibrosis stage are provided. The Jonckheere-Terpstra trend test was conducted to assess the trend (whether increasing or decreasing) of biomarker levels with fibrosis stage.

### Data

100 NASH Secretome candidate genes were generated after the bioinformatics filters were applied. Among these 100 targets, many code for proteins having functions related to NASH biology (Table 6).

**Table. 6. Secretome Candidates Participate in NASH Relevant Biological Pathways**

| **ECM** | **Membra ne receptors** | **Cytoskelet on myosin related** | **Neuroendocrin e** | **Immunoglobul in cyto/chemoki ne** | **Platelet/ compleme nt related** | **Enzyme/ TF** | **Others** |
|---|---|---|---|---|---|---|---|
| Col1A1 | EPHA3 | MYH10 | TENM4 | IGDCC4 | THBS2 | PLCE1 | FAM 129 B |
| Co13A1 | EPHB2 | MY05A | SYTL2 | IGSF3 | **C4BPB** | FUBP1 | FNDC1 |
| Co14A1 | FGFR2 | TPM1 | TANC1 | PRTG | **C4BPA** | AEBP1 | **HPX** |
| Col4A2 | FGFR3 | LIMA1 | NEO1 | SCF | **MASP2** | ZNF671 | **GC** |
| Co15A1 | DDR1 | CALD1 | ROBO1 | **CCL14** | **TFPI** | PTGDS | **AGT** |
| Col6A3 | TLR7 | PGDN | UNC5B | | | ZNF101 | **CPN2** |
| Col14A1 | LTBP2 | SPTAN1 | THY1 | | | LUZP1 | **CLEC3B** |
| CHI3L1 | MRC2 | KIF23 | NAV3 | | | ATP5J2 | **APOC3** |
| LOXL1 | ITGB6 | DPYSL3 | PCLO | | | FAP | **APOH** |
| LAMC2 | **MARCO** | TNS3 | LRRC4B | | | **CPB2** | **APCS** |
| EMILIN1 | **LYVE1** | UTRN | CADPS | | | **ALDH6A1** | |
| CDH6 | | | AHNAK | | | **PCBD1** | |
| PCDH18 | | | | | | **PRDX6** | |
| ADAMTSL 2 | | | | | | **PON3** | |
| ADAMS1 2 | | | | | | **SMPDL3A** | |
| AGRN | | | | | | **GLO1** | |
| DNAH7 | | | | | | **PSMB2** | |
| CCDC80 | | | | | | **CTH** | |
| YKL-40 | | | | | | **MOCS2** | |
| **FCN3** | | | | | | **SERPINF2** | |
| **FCN2** | | | | | | **KIAA1161** | |

| | | | | | | | |
|---|---|---|---|---|---|---|---|
| * bold and underlined: levels decreased with increased fibrosis stage; otherwise levels decreased with increased fibrosis stage. | | | | | | | |

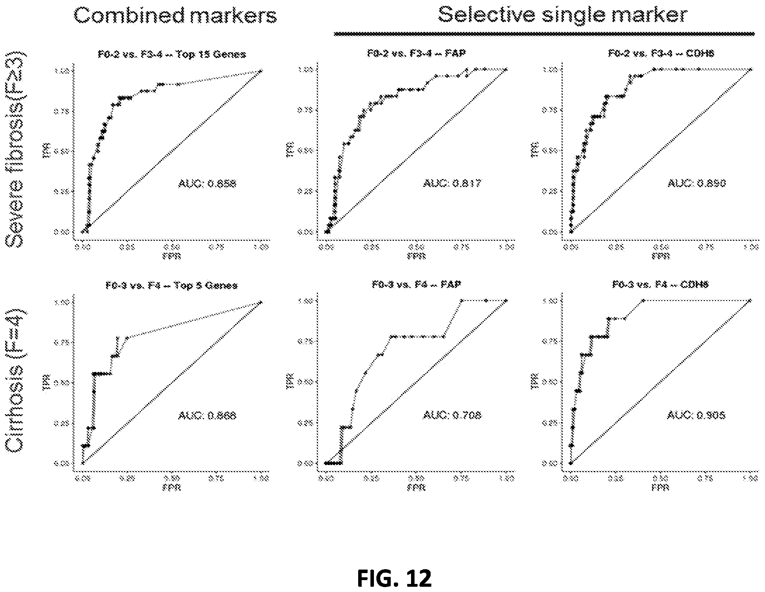

This example then performed analyses to ask whether hepatic expressions of these genes can be used as classifiers for severe fibrosis and/or cirrhosis. It was noticed that both single (FAP or CDH6) and combined genes (panel of 15 for F3, panel of 5 for F4) are good classifier for severe fibrosis or cirrhosis (FIG. 12).

However, utilizing hepatic RNA as NASH biomarkers is not practical, circulating protein levels of these candidates are further explored to be candidates for diagnostic and/or disease monitoring markers for fibrosis in NASH.

Two approaches were taken to generate circulating protein data for selected Secretome candidates, SOMAscan and ELISA.

### 1. SOMAscan

There are 23 Secretome candidates included in the SOMAscan platform.

**Table 7. Secretome Candidates Included in SOMAascan**

| **Protein** | **Gene** | **Full name** |
|---|---|---|
| TSP2 | THBS2 | Thrombospondin-2 |
| MRC2 | MRC2 | C-type mannose receptor 2 |
| SAP | APCS | Serum amyloid P-component |
| EPHB2 | EPHB2 | Ephrin type-B receptor 2 |
| SPTA2 | SPTAN1 | Spectrin alpha chain, non-erythrocytic 1 |
| A2AP | SERPINF2 | Alpha 2-antiplasmin |
| CDH6 | CDH6 | Cadherin-6 |
| CCDC80 (URB) | CCDC80 | Coiled-coil domain-containing protein 80 |
| CCL14 (HCC-1) | CCL14 | C-C motif chemokine 14 |
| CPB2 (TAFI) | CPB2 | Carboxypeptidase B2 |
| DDR1 | DDR1 | Epithelial discoidin domain-containing receptor 1 |
| EPHA3 | EPHA3 | Ephrin type-A receptor 3 |
| FCN2 | FCN2 | Ficolin-2 |
| FCN3 | FCN3 | Ficolin-3 |
| FGFR2 | FGFR2 | Fibroblast growth factor receptor 2 |
| FGFR3 | FGFR3 | Fibroblast growth factor receptor 3 |
| HPX | HPX | Hemopexin |
| KIF23 | KIF23 | kinesin family member 23provided |
| LYVE1 | LYVE1 | Lymphatic vessel endothelial hyaluronic acid receptor 1 |
| PRDX6 | PRDX6 | Peroxiredoxin 6 |
| ASM3A | SMPDL3A | Acid sphingomyelinase-like phosphodiesterase 3a |
| TPM1 | TPM1 | Tropomyosin 1 alpha chain |
| TFPI | TFPI | Tissue factor pathway inhibitor |

Circulating levels of the coded proteins for these 23 genes were examined from the SOMA logic dataset in Example 1 and it was found that:
- TSP2, MRC2, SAP, EPHB2, SPTA2, and SEPR were significantly (p < 0.05) associated with CRN fibrosis stage.
- No significant associations identified between changes in these markers with improvements in fibrosis stage, however, change in A2AP showed potential disease monitoring characteristics.
- Changes in TSP2, MRC2, SAP, EPHB2, HPX, and LYVE1 were associated with steatosis improvement (Table 8).

**Table 8. Changes in Secretome Candidate Markers with Improvements in Fibrosis or NAS Components in SOMAscan Dataset (1497; BL/Week 24)**

| | **Fibrosis** | **Steatosis** | **LI** | **HB** |
|---|---|---|---|---|
| THBS2(TSP2) | P=0.47 | P<0.001 | P<0.05 | P=0.085 |
| MRC2 | P=0.84 | P<0.05 | P=0.67 | P=0.41 |
| APCS(SAP) | P=0.44 | P<0.001 | P=0.25 | P=0.63 |
| EPHB2 | P=0.62 | P<0.05 | P=0.66 | P=0.67 |
| CPB2(TAFI) | P=0.52 | P=0.59 | P<0.05 | P=0.55 |
| DDR1(discoidin domain receptor 1) | P=0.51 | P=0.63 | P<0.05 | P=0.87 |
| HPX(hemopexin) | P=0.72 | P<0.005 | P=0.28 | P=0.24 |
| KIF23 | P=0.14 | P=0.30 | P<0.01 | P=0.93 |
| LYVE1 | P=0.41 | P<0.005 | P<0.01 | P=0.38 |

### 2. ELISA Assays

ELISA assays were developed and qualified for secretome candidates that were not included on the SOMAscan (ITGB6, FNDC1, MCM2, EMILIN1, IGDCC4, MASP2, SCF, LTBP2, ADAMTS12) as well as for those (TSP2, A2AP, MRC2, SAP, CTSH, IGFBP7, C7, MAC2BP) that were on SOMAscan platform and demonstrated promising preliminary results on fibrosis staging associations (Table 9).

**Table 9. ELISA Assays Developed for Secretome Candidates**

| **Protein** | **Gene** | **Full name** | **Biological function** |
|---|---|---|---|
| CHI3L1 (YKL-40) | CHI3L1 | Chitinase-3-like protein 1 | ECM glycoprotein |
| FAP | FAP | Fibroblast activation protein | Membrane-bound protease |
| ITGB6 | ITGB6 | Integrin, beta 6 | Membrane receptor |
| FNDC1 | FNDC1 | Fibronectin type III domain containing 1 | G-protein signaling |
| MCM2 | MCM2 | Mini-chromosome maintenance protein 2 | Genome replication/liver regeneration |
| EMILIN1 | EMILIN1 | Elastin microfibril interfacer 1 | ECM glycoprotein |
| IGDCC4 | IGDCC4 | Immunoglobulin superfamily DCC subclass member 4 | Immunoglobulin |
| MASP2 | MASP2 | Mannan-binding lectin serine protease 2 | Protease to cleave complement(C4/C2) |
| SCF(Kit ligand) | KITLG | Kit ligand | Cytokine |
| LTBP2 | LTBP2 | Latent-transforming growth factor beta-binding protein 2 | ECM; binds to TGF 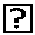 |
| ADAMTS12 | ADAMTS12 | A disintegrin and metalloproteinase with thrombospondin motifs 12 | Extracellular protease; promote fibrogenesis |
| TSP2 | THBS2 | Thrombospondin-2 | Glycoprotein; mediates cell-to- cell and cell-to-matrix interactions |
| A2AP | SERPINF2 | Alpha 2-antiplasmin | Serine protease inhibitor |
| MRC2 | MRC2 | C-type mannose receptor 2 | Endocytotic receptor; internalizes glycosylated ligands |
| SAP | APCS | Serum amyloid P-component | Acute phase proteins (APP) |
| CTSH | CTSH | Cathepsin H | lysosomal cysteine proteinase |
| IGFBP7 | IGFBP7 | Insulin-like growth factor-binding protein 7 | Controls availability of IGFs to tissue/cells |
| C7 | C7 | Complement C7 | Serum glycoprotein, complement complex component |
| MAC2BP | LGALS3BP | Galectin-3-binding protein | Modulates cell-cell and cell- matrix interactions |

### Results

### Association of Circulating Secretome Candidates with Fibrosis Stages

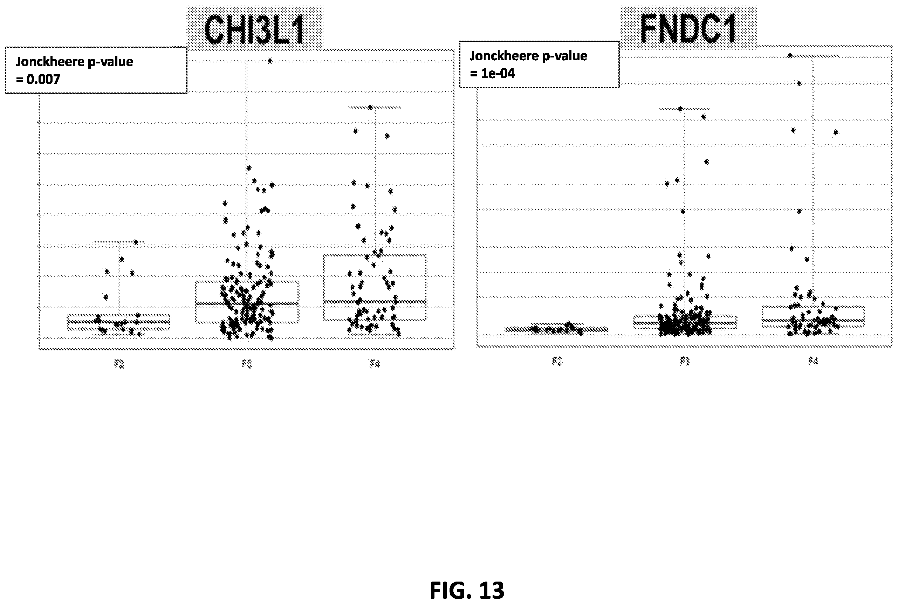

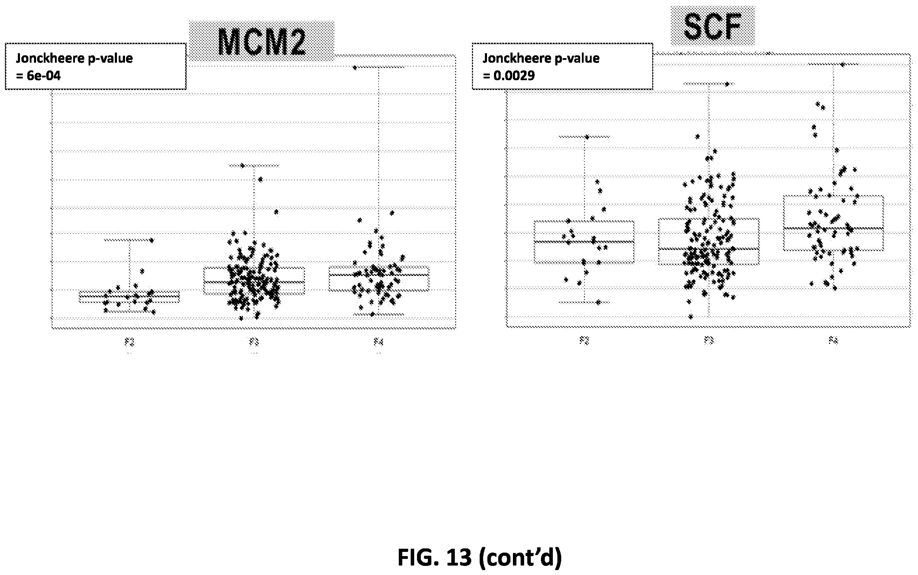

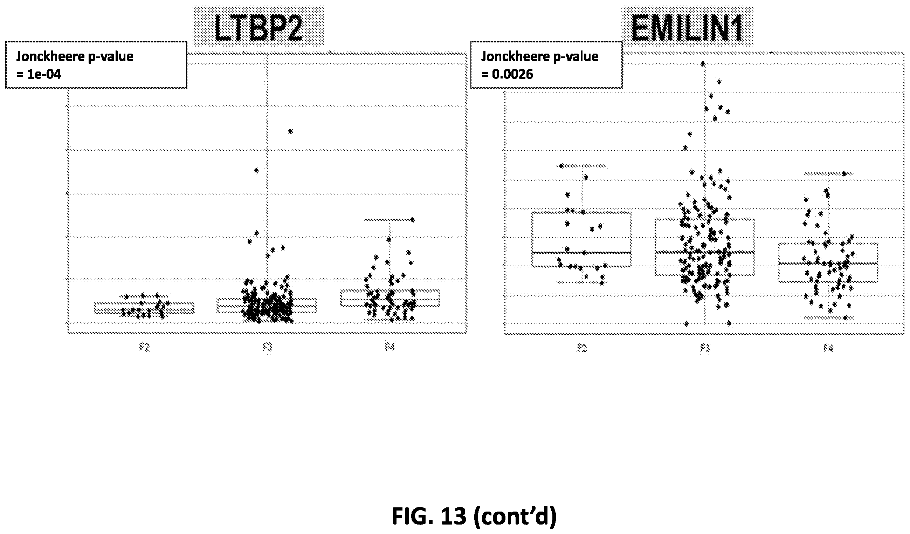

ELISA assays were performed in batches, first 11 candidates (CHI3L1, FAP, ITGB6, FNDC1, MCM2, EMILIN1, IGDCC4, MASP2, SCF, LTBP2, ADAMTS12) were tested and 6 out of 11 candidates demonstrated significant association with fibrosis stage (**FIG. 13**).

Due to limitation in 1497 study samples, 8 additional secretome candidates (TSP2, A2AP, MRC2, SAP, CTSH, IGFBP7, C7, MAC2BP) were examined using additional samples both at baseline and at week 48. 5 out of 8 candidates demonstrated significant association with fibrosis stage, including TSP2, A2AP, SAP, IGFBP7 and C7.

These results showed that the bioinformatics predictive algorism developed here successfully identified potential hepatic secreted/leaked proteins that correlate with NASH disease severity and could have potential for diagnosing and monitoring fibrosis in NASH.

### Diagnosis of CRN Fibrosis Stage (Advanced Fibrosis [F3, 4] and Cirrhosis [F4])

In order to evaluate the performance of these novel protein candidates on staging fibrosis in NASH subjects, univariate and multivariate analysis were used to identify individual markers or marker sets that can classify subjects with advanced fibrosis (F≥3) or with cirrhosis and the results are summarized below.

AUROC ≥ 0.7 was observed for diagnosing advanced fibrosis for IGFBP7 and TSP2, and for diagnosing cirrhosis for SAP, A2AP and IGFBP7 using *baseline and wk48* data [Table 16.8.2.1]. C7 had AUROC of 0.65 for diagnosing cirrhosis.

Differences were observed in AUROC for diagnosing baseline advanced fibrosis and cirrhosis using *baseline and screen fail* data and *baseline*, *screen fail and wk48* data due to small sample size in F0-2 in *baseline and screen fail* data (Table 10).

**Table 10. Evaluation of Secretome Panel for Diagnosing Baseline Advanced Fibrosis (F3-4 vs. F0-2) and Cirrhosis (F4 vs. F0-3) in Enrolled, Screen-Failed and Week 48 Data from Combined Studies**

| **Test** | **AUC (95% CI)*** | | | | | | |
|---|---|---|---|---|---|---|---|
| | **BL + SF (3 studies) F3, 4 *vs.* F0, 1, 2** | **BL + SF (3 studies) F4 *vs.* F0, 1, 2,3** | **Wk24 (1497) F3 *vs.* F1, 2** | **Wk48 (105/106) F3, 4 *vs.* F0, 1, 2** | **Wk48 (105/106) F4 *vs.* F0, 1, 2, 3** | **BL/SF + Wk48 (3 studies)** F3, 4 *vs.* F0, 1, 2** | **BL/SF + Wk48 (3 studies)** F4 *vs.* F0, 1, 2, 3** |
| ADAMTS12 | 0.54 (0.49, 0.57) | 0.53 (0.49, 0.55) | 0.52 (0.47, 0.62) | 0.58 (0.52, 0.65) | 0.53 (0.48, 0.55) | 0.51 (0.48, 0.55) | 0.54 (0.52, 0.55) |
| CHI3L1 | 0.66 (0.64, 0.68) | 0.55 (0.49, 0.57) | 0.52 (0.47, 0.59) | 0.59 (0.56, 0.62) | 0.63 (0.61, 0.64) | 0.63 (0.6, 0.64) | 0.6 (0.59, 0.61) |
| EMILIN1 | 0.53 (0.45, 0.58) | 0.62 (0.61, 0.62) | 0.58 (0.44, 0.67) | 0.59 (0.57, 0.61) | 0.56 (0.54, 0.57) | 0.57 (0.54, 0.59) | 0.6 (0.59, 0.6) |
| FAP | 0.55 (0.48, 0.6) | 0.51 (0.48, 0.57) | 0.66 (0.63, 0.68) | 0.64 (0.62, 0.65) | 0.71 (0.69, 0.71) | 0.54 (0.51, 0.56) | 0.58 (0.57, 0.58) |
| FNDC1 | 0.81 (0.8, 0.81) | 0.57 (0.51, 0.59) | 0.58 (0.56, 0.61) | 0.58 (0.48, 0.66) | 0.54 (0.47, 0.61) | 0.56 (0.53, 0.59) | 0.55 (0.52, 0.57) |
| IGDCC4 | 0.57 (0.54, 0.62) | 0.52 (0.48, 0.58) | 0.6 (0.51, 0.69) | 0.53 (0.47, 0.58) | 0.52 (0.47, 0.56) | 0.51 (0.48, 0.56) | 0.52 (0.48, 0.55) |
| ITGB6 | 0.56 (0.48, 0.68) | 0.54 (0.5, 0.57) | 0.55 (0.46, 0.67) | 0.51 (0.44, 0.55) | 0.57 (0.54, 0.59) | 0.52 (0.48, 0.59) | 0.56 (0.54, 0.57) |
| MASP2 | 0.59 (0.56, 0.65) | 0.53 (0.48, 0.58) | 0.59 (0.47, 0.69) | 0.53 (0.48, 0.58) | 0.52 (0.48, 0.56) | 0.52 (0.49, 0.57) | 0.52 (0.49, 0.55) |
| SCF | 0.55 (0.47, 0.64) | 0.63 (0.62, 0.64) | 0.55 (0.46, 0.66) | 0.59 (0.5, 0.66) | 0.6 (0.57, 0.61) | 0.57 (0.52, 0.63) | 0.63 (0.62, 0.63) |
| LTBP2 | 0.62 (0.6, 0.63) | 0.65 (0.63, 0.67) | 0.59 (0.53, 0.63) | 0.53 (0.47, 0.59) | 0.54 (0.48, 0.61) | 0.58 (0.53, 0.61) | 0.53 (0.48, 0.58) |
| MCM2 | 0.76 (0.74, 0.77) | 0.58 (0.56, 0.59) | 0.52 (0.45, 0.64) | 0.51 (0.48, 0.56) | 0.56 (0.54, 0.58) | 0.57 (0.55, 0.58) | 0.59 (0.58, 0.59) |
| FNDC1 + MCM2 | 0.84 (0.83, 0.86) | 0.6 (0.57, 0.61) | 0.54 (0.43, 0.61) | 0.55 (0.51, 0.6) | 0.53 (0.47, 0.56) | 0.57 (0.55, 0.59) | 0.59 (0.58, 0.6) |
| FNDC1 + MCM2 + BA + NFS | 0.86 (0.83, 0.87) | 0.77 (0.75, 0.78) | 0.65 (0.59, 0.7) | 0.78 (0.75, 0.8) | 0.8 (0.77, 0.82) | 0.72 (0.7, 0.74) | 0.8 (0.79, 0.81) |

| **Test** | **AUC (95% CI)*** | | | | | | |
|---|---|---|---|---|---|---|---|
| | **BL F3, 4 *vs.* F2** | **BL F4 *vs.* F3** | **Wk24 (1497) F3 *vs.* F1, 2** | | | **BL + Wk48 F3, 4 *vs.* F0, 1, 2** | **BL + Wk48 F4 *vs.* F0, 1, 2, 3** |
| TSP2 | | 0.63 (0.61, 0.64) | | 0.7 (0.69, 0.72) | 0.71 (0.7, 0.72) | 0.71 (0.69, 0.72) | 0.67 (0.66, 0.67) |
| A2AP | | 0.71 (0.7, 0.72) | | 0.67 (0.65, 0.68) | 0.72 (0.71, 0.72) | 0.66 (0.64, 0.66) | 0.71 (0.71, 0.72) |
| MRC2 | | 0.58 (0.47, 0.65) | | 0.59 (0.47, 0.65) | 0.52 (0.47, 0.58) | 0.58 (0.47, 0.65) | 0.52 (0.47, 0.58) |
| SAP | | 0.78 (0.77, 0.78) | | 0.62 (0.59, 0.63) | 0.77 (0.76, 0.77) | 0.59 (0.56, 0.61) | 0.77 (0.77, 0.78) |
| CTSH | | 0.5 (0.48, 0.53) | | 0.55 (0.52, 0.58) | 0.52 (0.43, 0.57) | 0.52 (0.47, 0.55) | 0.51 (0.46, 0.55) |
| IGFBP7 | | 0.63 (0.6, 0.64) | | 0.76 (0.75, 0.77) | 0.78 (0.77, 0.78) | 0.75 (0.74, 0.76) | 0.71 (0.7, 0.71) |
| C7 | | 0.72 (0.71, 0.73) | | 0.54 (0.5, 0.56) | 0.56 (0.53, 0.57) | 0.53 (0.49, 0.58) | 0.65 (0.64, 0.65) |
| MAC2BP | | 0.54 (0.49, 0.59) | | 0.6 (0.58, 0.62) | 0.61 (0.59, 0.62) | 0.59 (0.56, 0.61) | 0.55 (0.53, 0.56) |

| | | | | | | | |
|---|---|---|---|---|---|---|---|
| * Denotes mean and corresponding 95% CI for 5-fold cross-validation repeated 100 times. * * BL/SF from all 3 studies, but wk48 values from only SIM 105/106 (analyzing all data together). | | | | | | | |

### Monitoring of CRN Fibrosis Stage Change (Improvement or Worsening)

This example next examined the longitudinal changes of these novel protein markers and asked whether these markers can have potential values for monitoring fibrosis stage changes and the results are summarized below.

Using only % *change from baseline* as predictors, several markers have AUROC between 0.60-0.70 for monitoring fibrosis stage changes but none of the markers from secretome panel have performance of AUROC ≥ 0.7 for *monitoring* CRN fibrosis improvement or CRN fibrosis worsening.

In general, the performance of AUROC is improved when using *both baseline and %change from baseline* compared to using only change from baseline as predictors. FAP, LTBP2, SAP, IGFBP7, and MAC2BP have AUROC>0.70 for *monitoring* CRN fibrosis improvement or CRN fibrosis worsening (SIM 105).

### Prognosing of CRN Fibrosis Stage Change (Improvement or Worsening at Week 48) Using Baseline Biomarker Levels

This example also performed analysis to address whether baseline levels of these markers has prognosis values to predict week 48 fibrosis stage changes in SIM study samples since it is believed these studies are considered as nature progression due to lack of efficacy by SIM. The results are summarized below.

Several markers (using baseline levels) have performance of AUROC ≥ 0.7 for *predicting.*

CNR fibrosis improvement: IGFBP7 (F3 to lower at wk48 in SIM105, F4 to lower at wk48 in SIM106, F4/3 to lower at wk48), FAP and SCF (F4 to lower at wk48).

CRN fibrosis worsening (F3 to F4 at wk48): IGFBP7 and SAP.

### Monitoring of Changes (Improvement or Worsening) in NAS Score Components (not according to the invention)

Using *%change from baseline* as predictors, several markers have AUROC between 0.60-0.70 for *monitoring* improvement in NAS component (hepatic ballooning, lobular inflammation or steatosis).

In general, the performance of AUROC for *monitoring* improvement in NAS component is improved when using *both baseline and change from baseline* as predictors. AUROC ≥ 0.7 was observed for FAP for monitoring lobular inflammation improvement (Grade 3 to lower at wk48), and ADAMTS 12 for monitoring steatosis improvement (Grade 2/3 to lower at wk48).

### Diagnosis of NASH vs. Non-NASH in SEL 1497, SIM 0105 and 0106 Studies (not according to the invention)

Using data from baseline and wk48, TSP2 had AUROC of 0.66/0.68/0.7/0.71 for *diagnosing* NASH vs. non-NASH [4 definitions of non-NASH: 1) 0 for any NAS subscore (lobular inflammation, hepatic ballooning or steatosis); 2) no to mild inflammation; 3) no ballooning; 4) no active NASH (no to mild inflammation and no ballooning)]. Similar AUROC was observed when using only baseline data from enrolled patients in combined SIM105/106 studies.

### Conclusions

In this example, a new approach was taken to generate potential liver selective and fibrosis stage dependent protein biomarkers using biopsy derived transcriptome data and bioinformatic algorisms to predict secreted/leaking proteins. This strategy was proven to be fruitful with the following key findings, (1) candidate genes code for proteins that are involved in fibrosis biology; (2) hepatic expression profiles of these genes (single or selective panel) have good classifying characteristics (AUROC 0.8-0.9) for identification of severe fibrosis (F3) or cirrhosis (F4); and (3) circulating levels of selective candidates were evaluated either by SOMAscan (depends on availability) or ELISA assays.

AUROC ≥ 0.7 was observed for diagnosing advanced fibrosis for IGFBP7 and TSP2, and for diagnosing cirrhosis for SAP, A2AP and IGFBP7 using *baseline, screen fail and wk48* data. C7 had AUROC of 0.65 for diagnosing cirrhosis.

None of the markers from secretome panel (using *%change from baseline*) had AUROC ≥ 0.7 for monitoring CRN fibrosis changes (either improvement or worsening) and improvement in NAS component (hepatic ballooning, lobular inflammation, and steatosis). In general, the performance of AUROC is improved when using *both baseline and %change from baseline.*

Using *baseline levels,* several markers had AUROC ≥ 0.7 for predicting:
CRN fibrosis improvement: IGFBP7 (F3 to lower at wk48, F4 to lower at wk48, F4/3 to lower at wk48), FAP and SCF (F4 to lower at wk48); and
Fibrosis worsening (F3 to F4 at wk48): IGFBP7 and SAP.

Using data from baseline and wk48 in combined SIM105/106 studies, TSP2 had AUROC of ~0.7 for *diagnosing* NASH vs. non-NASH across the 4 definitions.

### Example 4. Additional SOMAscan Data for GDF-15 and CD163 (not according to the invention)

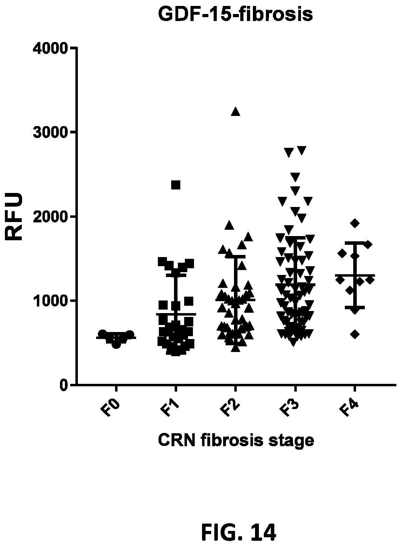

This example reports additional SOMAscan data collected with the method in Example 1, for the circulating proteins GDF-15 and CD163. Summary data are presented in charts in **FIG. 14-18****.**

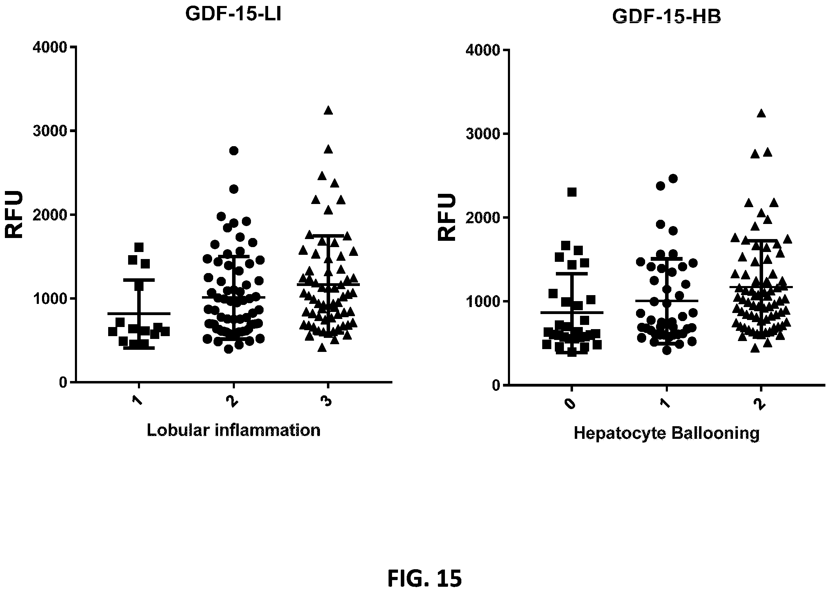

As shown in **FIG. 14****,** circulating GDF-15 levels were significantly associated with fibrosis stage in NASH subjects; p<0.0001 (Kruskal-Wallis test). Likewise, as shown in **FIG. 15****,** the circulating GDF-15 levels were significantly associated with Lobular inflammation (left panel; P<0.05 (Kruskal-Wallis test)) and Hepatic Ballooning in the NASH subjects (right panel; P<0.005 (Kruskal-Wallis test)). However, the circulating GDF-15 levels were not associated with steatosis or NAS scores in the NASH subjects.

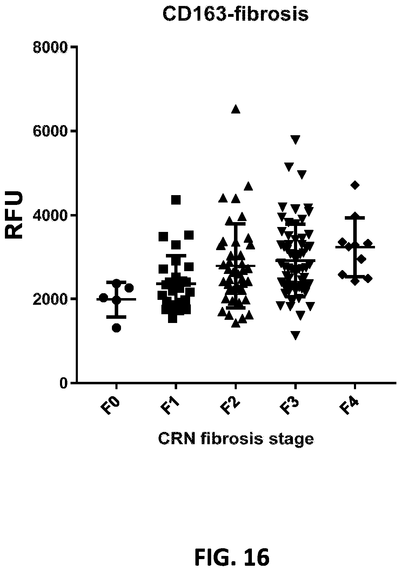

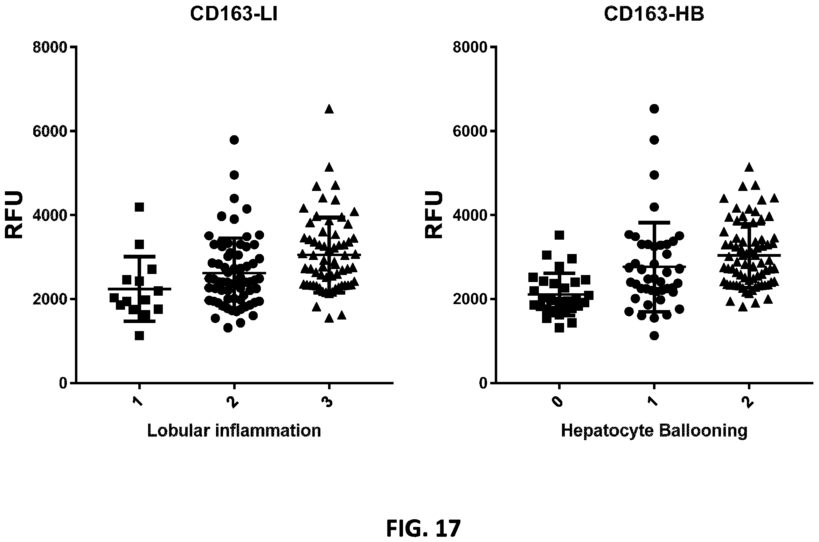

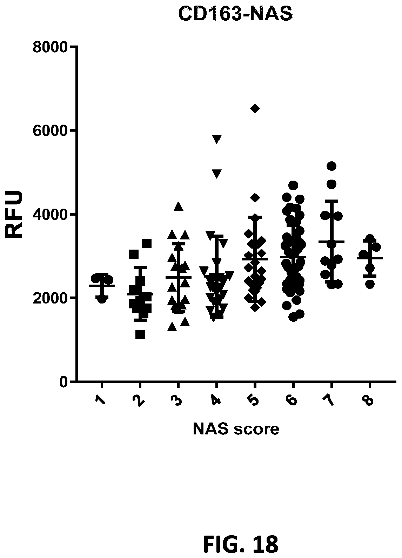

The chart in **FIG. 16** shows that circulating CD163 levels were significantly associated with fibrosis stages in NASH subjects, p<0.001 (Kruskal-Wallis test). The circulating CD163 levels were also significantly associated with Lobular inflammation **(****FIG. 17****,** left panel, p<0.0005 (Kruskal-Wallis test)) and Hepatic Ballooning **(****FIG. 17****,** right panel, p<0.0001 (Kruskal-Wallis test)) in the NASH subjects. The circulating CD163 levels were also significantly associated with NAS scores **(****FIG. 18****,** p<0.001 (Kruskal-Wallis test)) but not with steatosis in the NASH subjects.

The results of Examples 3 and 4 are summarized in the following tables, complementary to Tables 1-4.

### Tables 11A-D: Protein markers for diagnosis

**Table 11A. Protein Markers for CRN Fibrosis Stages (CRN)**

| **Marker Name** | **UniProt** | **p value** | **CRN fibrosis stage 0** | **CRN fibrosis stage 1** | **CRN fibrosis stage 2** | **CRN fibrosis stage 3** | **CRN fibrosis stage 4** |
|---|---|---|---|---|---|---|---|
| YKL-40 | P36222 | <0.01 | N/A | N/A | 63100 | 124716 | 129864 |
| FAP | Q12884 | 0.606 | N/A | N/A | 50761 | 44068 | 44705 |
| ITGB6 | P18564 | 0.202 | N/A | N/A | 14.5 | 14.0 | 17.1 |
| EMILIN1 | Q9Y6C2 | <0.005 | N/A | N/A | 16802 | 16818 | 14941 |
| FNDC1 | Q4ZHG4 | <0.0005 | N/A | N/A | 0.4 | 1.0 | 1.2 |
| IGDCC4 | Q8TDY8 | 0.076 | N/A | N/A | 321.2 | 274.1 | 262 |
| MASP2 | 000187 | 0.127 | N/A | N/A | 718.8 | 579.5 | 591.8 |
| SCF | P21583 | <0.005 | N/A | N/A | 732.3 | 693.7 | 798.8 |
| LTBP2 | Q14767 | <0.0005 | N/A | N/A | 1.5 | 1.9 | 2.6 |
| ADAMTS12 | P58397 | 0.199 | N/A | N/A | 15825.8 | 19191 | 25317.7 |
| MCM2 | P49736 | <0.0005 | N/A | N/A | 12.1 | 16.1 | 18.1 |
| GDF-15 | Q99988 | <0.0001 | 569.4 | 657 | 917 | 1046 | 1247 |
| CD163 | Q86VB7 | <0.001 | 2027 | 2190 | 2615 | 2746 | 3265 |

**Table 11B. Protein Markers for NAS Scores (NAS) (not according to the invention)**

| **Marker Name** | **UniProt** | **p value** | **NAS stages** | | | | | | | |
|---|---|---|---|---|---|---|---|---|---|---|
| CD163 | Q86VB7 | <0.001 | 2429 | 1943 | 2363 | 2258 | 2676 | 2817 | 2918 | 3033 |

**Table 11C. Protein Markers for Lobular Inflammation (LI) (not according to the invention)**

| **Marker Name** | **UniProt** | **p value** | **Lobular inflammation stage 0** | **Lobular inflammation stage 1** | **Lobular inflammation stage 2** |
|---|---|---|---|---|---|
| GDF-15 | Q99988 | <0.05 | 623.2 | 858.6 | 1024 |
| CD163 | Q86VB7 | <0.001 | 2002 | 2413 | 2846 |

**Table 11D. Protein Markers for Hepatic Ballooning (HB) (not according to the invention)**

| **Marker Name** | **UniProt** | **p value** | **Hepatic ballooning stage 0** | **Hepatic ballooning stage 1** | **Hepatic ballooning stage 2** |
|---|---|---|---|---|---|
| GDF-15 | Q99988 | <0.005 | 630.9 | 774.7 | 1039 |
| CD163 | Q86VB7 | <0.0001 | 1966 | 2473 | 2863 |

### Table 12: Protein markers for monitoring clinical improvements (not according to the invention)

**Table 12. Protein Biomarkers for Monitoring Improvements in NASH related clinical endpoints**

| **Marker Name** | **UniProt** | **Clinical endpoints** | **Median percent CHG at W24 in Non-improver** | **Median percent CHG at W24 in improver** | **p value** |
|---|---|---|---|---|---|
| YKL-40 | P36222 | NAS score | 0.5 | -23.9 | <0.05 |
| YKL-40 | P36222 | Steatosis | 10.8 | -22.9 | <0.05 |
| FAP | Q12884 | Steatosis | 2.4 | -6.2 | <0.05 |
| MCM2 | P49736 | Steatosis | 4.3 | -11.8 | 0.08 |
| SCF | P21583 | Ballooning | 1.9 | 7.4 | <0.05 |
| YKL-40 | P36222 | Inflammation | 4.3 | -22 | <0.05 |
| FAP | Q12884 | Inflammation | 1.5 | -12.6 | <0.05 |
| MCM2 | P49736 | MRE | 4.3 | -7.9 | 0.06 |
| YKL-40 | P36222 | MRIPDFF | 6.5 | -22 | <0.05 |
| EMILIN1 | Q9Y6C2 | MRIPDFF | -2.8 | -16.6 | <0.05 |
| FAP | Q12884 | MRIPDFF | 1.2 | -17.5 | <0.05 |

### Example 5. Algorithms Using Noninvasive Tests Can Accurately Identify Patients with Advanced Fibrosis due to NASH: Data from STELLAR Clinical Trials (not according to the invention)

There is a major unmet need for accurate, readily available noninvasive tests (NITs) to identify patients with advanced fibrosis (F3-F4) due to NASH. The goal of this example was to evaluate sequential NITs to minimize the requirement for biopsy and improve accuracy over use of single tests.

**Methods:** The STELLAR studies (NCT03053050 and NCT03053063) enrolled NASH patients with bridging fibrosis (F3) or compensated cirrhosis (F4). Baseline liver biopsies were centrally read using the NASH CRN fibrosis classification and noninvasive markers of fibrosis, including the Fibrosis-4 (FIB-4) index, Enhanced Liver Fibrosis (ELF) test, and FibroScan^{®} (FS) were measured. The performance of these tests to discriminate advanced fibrosis was evaluated using AUROCs with 5-fold cross-validation repeated 100x. Thresholds were obtained by maximizing specificity given ≥85% sensitivity (and vice versa). The cohort was divided (80%/20%) into evaluation/validation sets. The evaluation set was further stratified 250x into training and test sets (66%/33%). Optimal thresholds were derived as average across training sets, and applied sequentially (FIB-4 followed by ELF and/or FS) to the validation set.

**Results:** All screened and enrolled patients with available liver histology (N=3202, 71% F3-F4) and NIT results were included in the analysis. Using thresholds derived from STELLAR study data, FIB-4 followed by FS or ELF test in those with indeterminate FIB-4 values (1.23 to 2.1) demonstrated good performance characteristics while minimizing frequency of indeterminate values to as low as 13% (Table). Using published NIT thresholds resulted in similar findings (data not shown). Adding a third test (FIB-4 then ELF then FS) reduced the rate of indeterminate results to 8%. Misclassification occurred at rates similar to biopsy (15-21%). The majority of misclassifications (63-81%) were false negatives; among false positive cases (19-27% of misclassifications) up to 70% had F2 fibrosis.

**Conclusion:** In these large, global, phase 3 trials with newly derived thresholds optimized for the STELLAR trials, FIB-4 followed by ELF and/or FS nearly eliminated the need for liver biopsy and accurately identified patients with advanced fibrosis due to NASH with misclassification rates similar to liver biopsy. Further validation of these findings in additional cohorts is planned.

**Table 13. Diagnostic Performance of NITs to Discriminate Advanced Fibrosis (F3-F4)**

| **Test*** | **Cohort** | **Sample Size** | **Sensitivity** | **Specificity** | **Indeterminate** | **Misclassified**** |
|---|---|---|---|---|---|---|
| FIB-4(1.23, 2.1) | Train+Test | 2496 | 85% | 85% | 32% | 15% |
| | Validation | 627 | 83% | 89% | 32% | 15% |
| ELF(9.35, 10.24) | Train+Test | 2536 | 85% | 85% | 29% | 15% |
| | Validation | 637 | 85% | 85% | 29% | 15% |
| FS(9.6kPa, 14.53kPa) | Train+Test | 1408 | 85% | 86% | 28% | 15% |
| | Validation | 357 | 82% | 88% | 25% | 17% |
| FIB-4(1.23, 2.1) then ELF(9.35, 10.24) | Train+Test | 2542 | 79% | 81% | 13% | 20% |
| | Validation | 638 | 78% | 82% | 13% | 21% |
| FIB-4(1.23, 2.1) then FS(9.6kPa, 14.53kPa) | Train+Test | 2509 | 82% | 85% | 20% | 17% |
| | Validation | 632 | 78% | 87% | 20% | 19% |

| | | | | | | |
|---|---|---|---|---|---|---|
| *Lower value represents optimal threshold to exclude advanced fibrosis, higher value to diagnose advanced fibrosis, with in-between values classified as indeterminate * *Proportion of misclassified patients relative to total sample size including indeterminate zone | | | | | | |

### Example 6. Identification of NASH associated serum metabolites and diagnosis biomarkers using an OWLiver^{®} metabolomics platform (not according to the invention)

This example was conducted to identify serum metabolites correlated with NASH disease severities, and to evaluate the performance of selected metabolite panels as classifiers for advance fibrosis, cirrhosis, active NASH and cryptogenic cirrhosis. The technology used here, referred to as OWLiver^{®} metabolomics, is commercially available from OWL Metabolomics (Bizkaia, Spain) and is described in Barr et al, J Proteome Res. 2010 Sep 3;9(9):4501-12 and Mayo et al., Hepatol Commun. 2018 May 4;2(7):807-820.

Study design: Samples: 279 serum samples from either healthy individuals or NAFLS/NASH subjects with various degree of biopsy-confirmed liver fibrosis (F0-F4) were included in current study (Table 14).

**Table 14. Serum samples included in the study**

| **Fibrosis stage** | **Healthy** | **F0** | **F1** | **F2** | **F3** | **F4** | **Total** |
|---|---|---|---|---|---|---|---|
| No. of samples | 30 | 39 | 45 | 55 | 52 | 58 | 279 |

Assay platform: Metabolomics profiling in serum were performed using mass spectrometry (MS) based approach at OWL Metabolomics.

Data analysis: Trend analysis was performed for categorical NASH parameters(fibrosis, NAS & components) and correlation analysis was performed for continuous variables(MQC, ELF, MRE, and MRI-PDFF). Univariate logistic regression (LR) with 5-fold cross-validation 100 times was used to select markers with AUROC>=0.7. Wilcoxon rank sum test was used to select markers with BH-adjusted p-value<0.05. Overlapping markers from the two analyses were selected as classifiers.

The analysis included metabolites in the families as shown in Table 15.

| **Family** | | **Number** |
|---|---|---|
| Phospho-ethanolamine (PE) | MEMAPE | 6 |
| | MAPE | 18 |
| | MEPE | 2 |
| | DAPE | 8 |
| Phospho-cholines (PC) | MAPC | 35 |
| | MEPC | 12 |
| | DAPC | 27 |
| | MEMAPC | 18 |
| Phospho-inositol (PI) | DAPI | 2 |
| | MAPI | 5 |
| | TAG | 50 |
| | DAG | 6 |
| | FFA | 21 |
| | Bile acids | 8 |
| | Acylcarnitines | 2 |
| | Ceramides | 3 |
| | Cholesterol Esters | 10 |
| | Sphingolipids | 22 |
| | Amino acids | 24 |
| | TAG | 50 |

**MEMAPE** (1-ether, 2-acylglycerophosphoethanolamine), **MAPE** (monoacylglycerophosphoethanolamine), **MEPE** (1-monoetherglycerophosphoethanolamine), **DAPE** (diacylglycerophosphoethanolamines), **MAPC** (1-monoacylglycerophosphocholine), **MEPC** (1-monoetherglycerophosphocholine), **DAPC** (diacylglycerophosphocholines), **MEMAPC** (1-ether, 2-acylglycerophosphocholine), **DAPI** (diacylglycerophosphoinositol), **MAPI** (monoacylglycerophosphoinositol), **TAG:** (triglycerides); **DAG** (diacylglycerides); **FFA** (free fatty acids)

Metabolites significantly associated with one or more of the fibrosis stages are listed in the tables below.

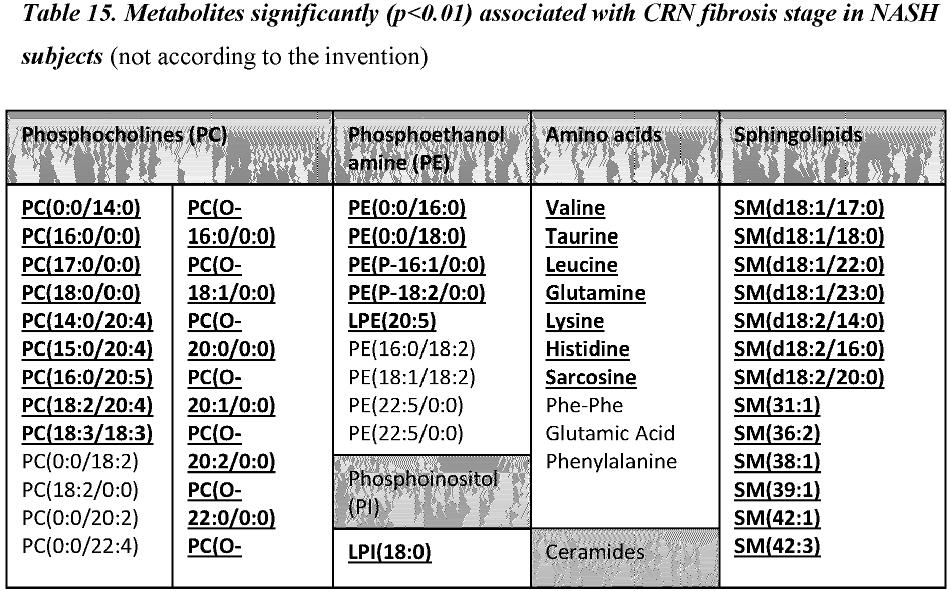

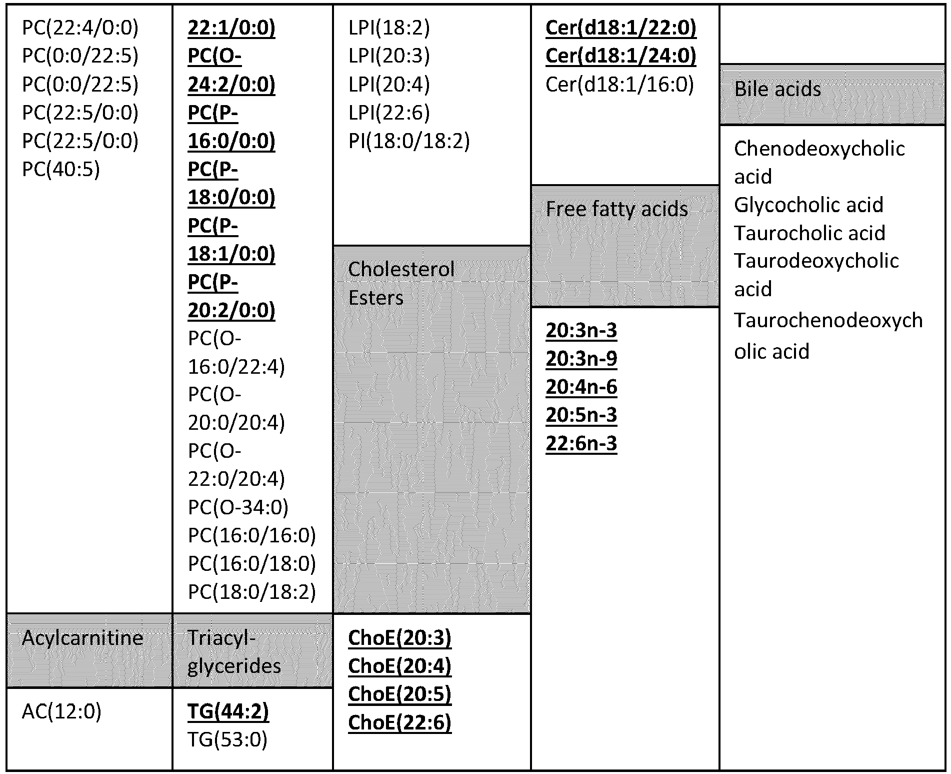

Underlined and bold: negative correlation; otherwise positive correlation.

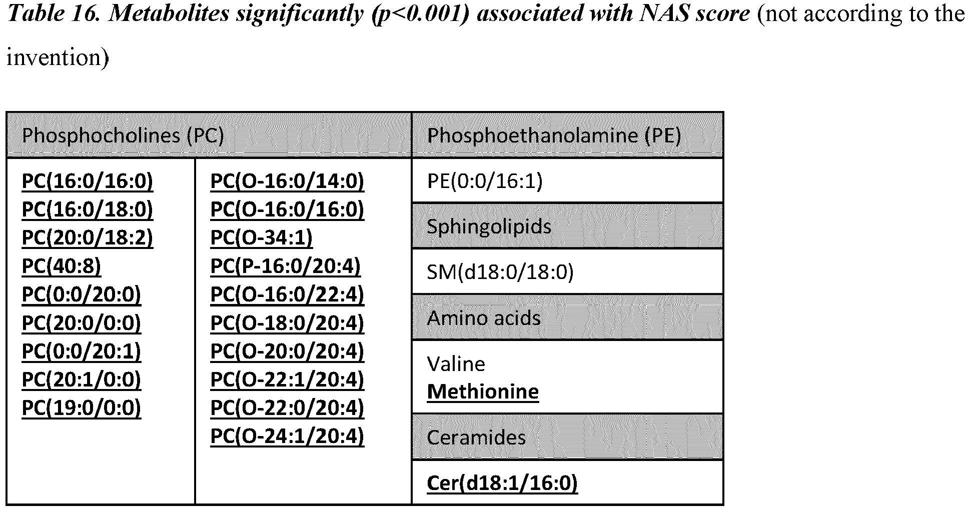

Underlined and bold: negative correlation; otherwise positive correlation.

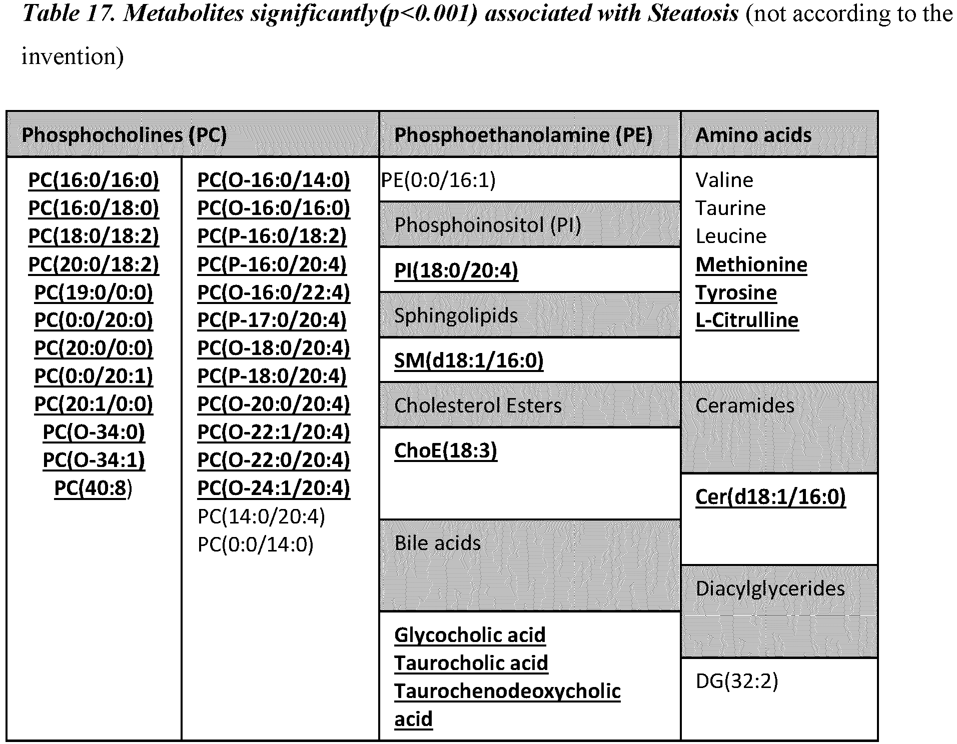

Underlined and bold: negative correlation; otherwise positive correlation.

**Table 18. Metabolites significantly (p<0.001) associated with Hepatocyte Ballooning (not according to the invention)**

| Phosphocholines (PC) | |
|---|---|
| **PC(O-22:1/20:4)** | **PC(20:0/18:2)** |
| **PC(O-24:1/20:4)** | **PC(O-16:0/14:0)** |
| **PC(19:0/0:0)** | **PC(40:8)** |
| **PC(O-34:1)** | **PC(0:0/20:0)** |
| **PC(O-22:0/20:4)** | **PC(O-16:0/20:4)** |
| **PC(O-20:0/20:4)** | **PC(O-18:0/20:4)** |
| **PC(O-16:0/16:0)** | **PC(16:0/18:0)** |
| **PC(P-16:0/20:4)** | |

Underlined and bold: negative correlation; otherwise positive correlation.

**Table 19. Metabolites significantly (p<0.01) associated with Lobular inflammation (not according to the invention)**

| Sphingolipids |
|---|
| SM(38:0) |
| SM(d18:0/18:0) |
| SM(d18:0/22:0) |

Underlined and bold: negative correlation; otherwise positive correlation.

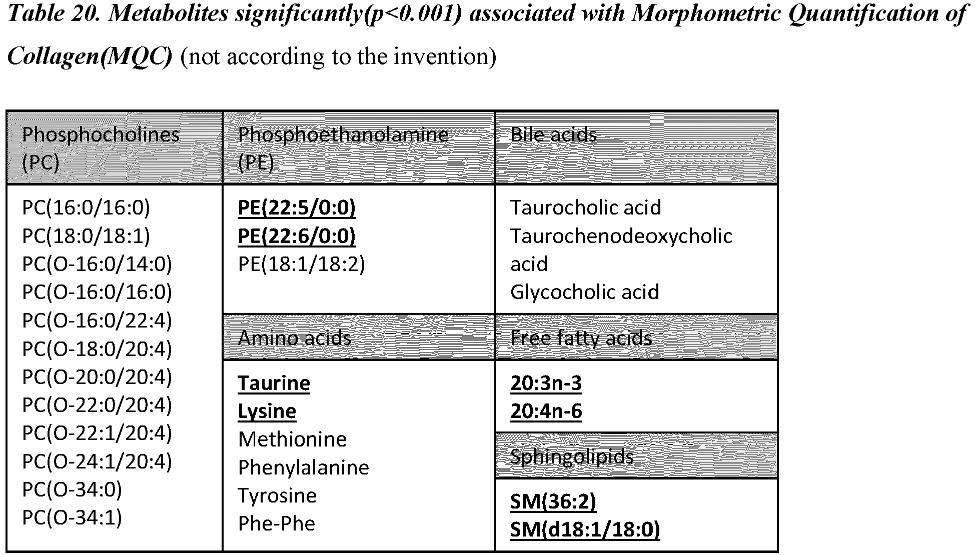

Underlined and bold: negative correlation; otherwise positive correlation.

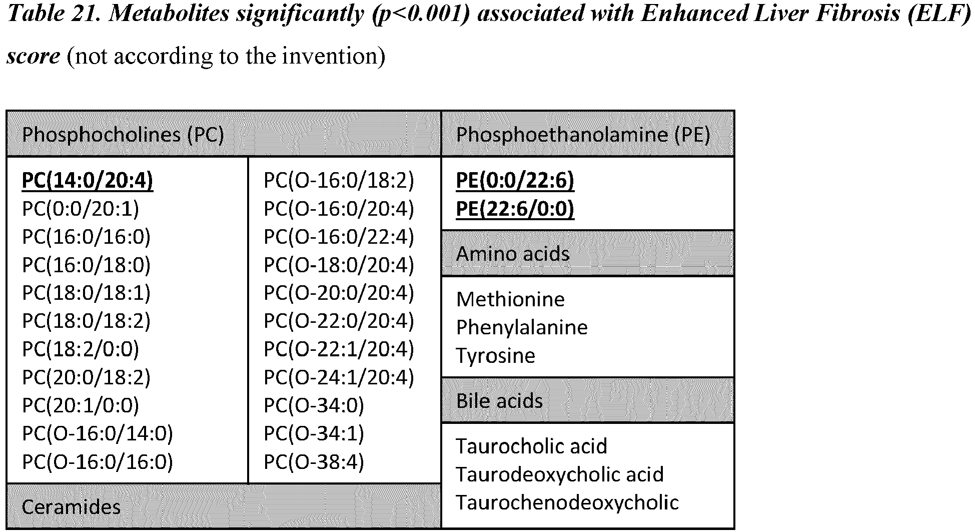

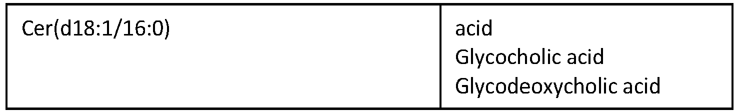

Underlined and bold: negative correlation; otherwise positive correlation.

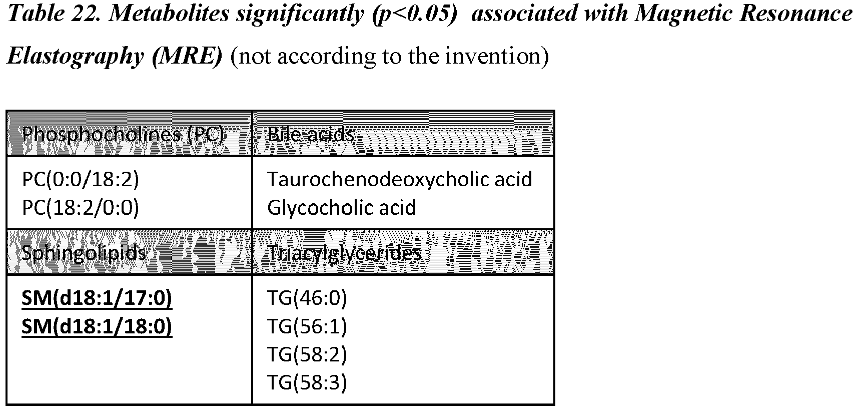

Underlined and bold: negative correlation; otherwise positive correlation.

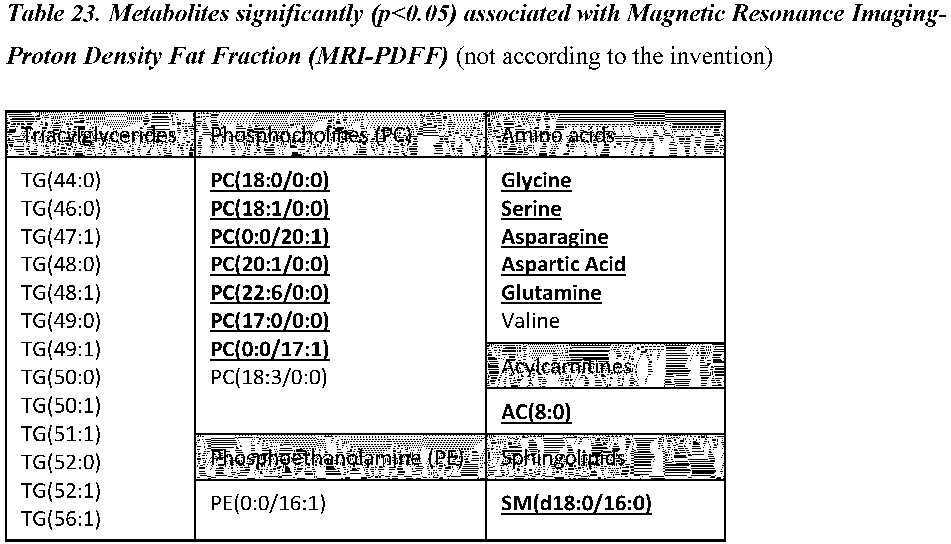

Underlined and bold: negative correlation; otherwise positive correlation.

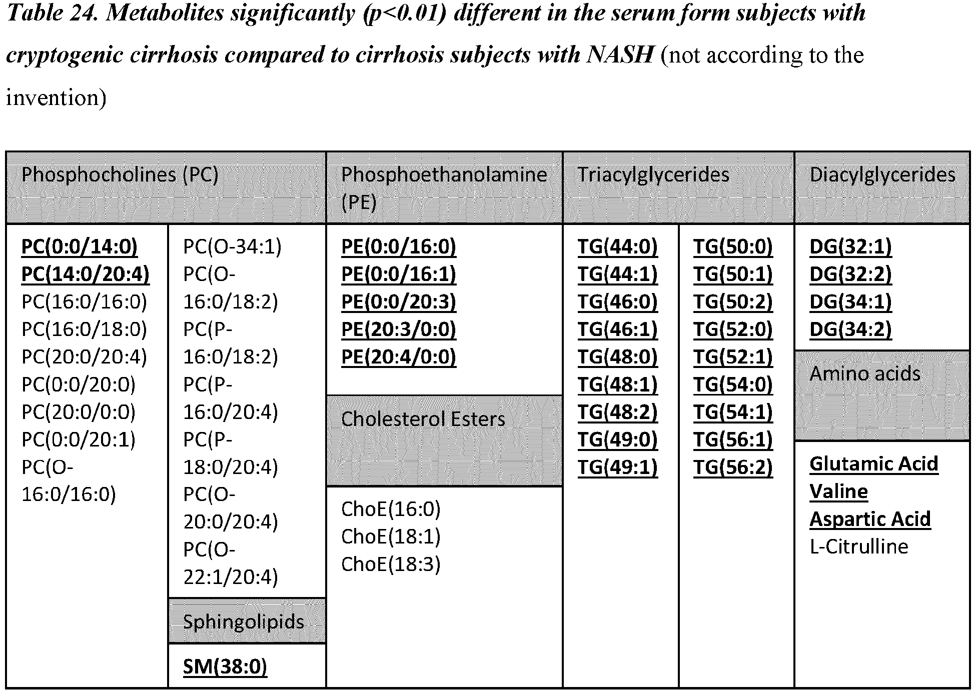

Underlined and bold: negative correlation; otherwise positive correlation.

**FIG. 19** shows common metabolite markers are present between different NASH phenotypes. Advance fibrosis (F>=3) alone: 17 metabolites; cirrhosis (F=4) alone: 30 metabolites; cryptogenic cirrhosis (F=4; no steatosis) alone: 34 metabolites; active NASH NAS score>=5 alone: 29 metabolites.

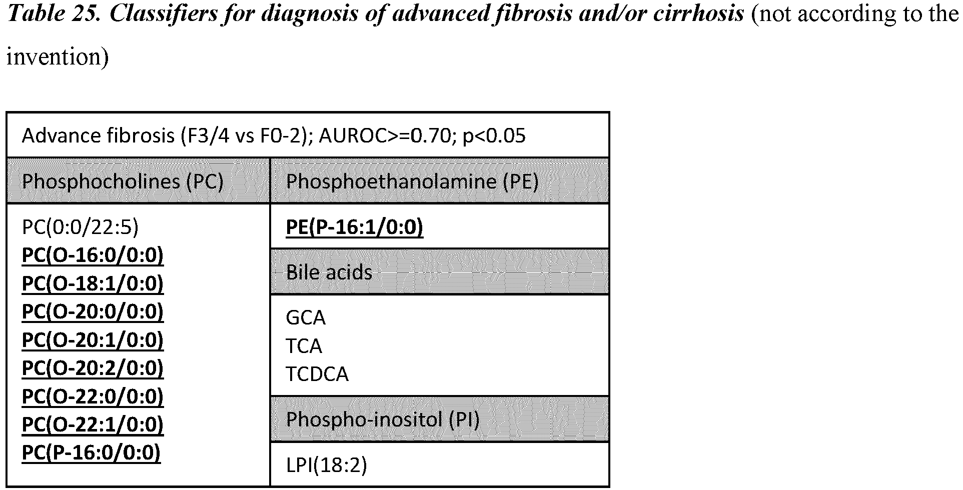

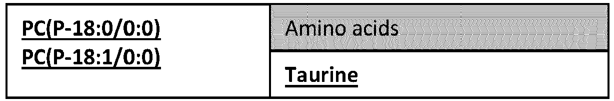

Underlined and bold: lower in F3/4; otherwise higher in F3/4

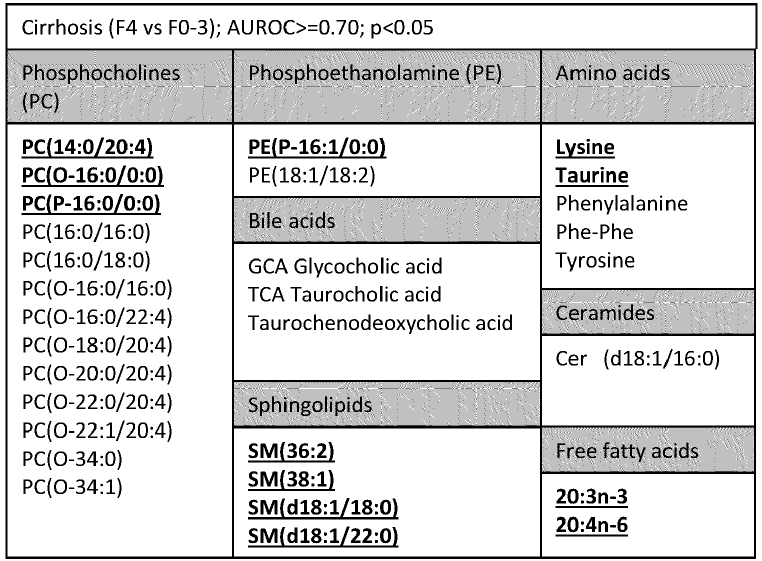

Underlined and bold: lower in F3/4; otherwise higher in F3/4

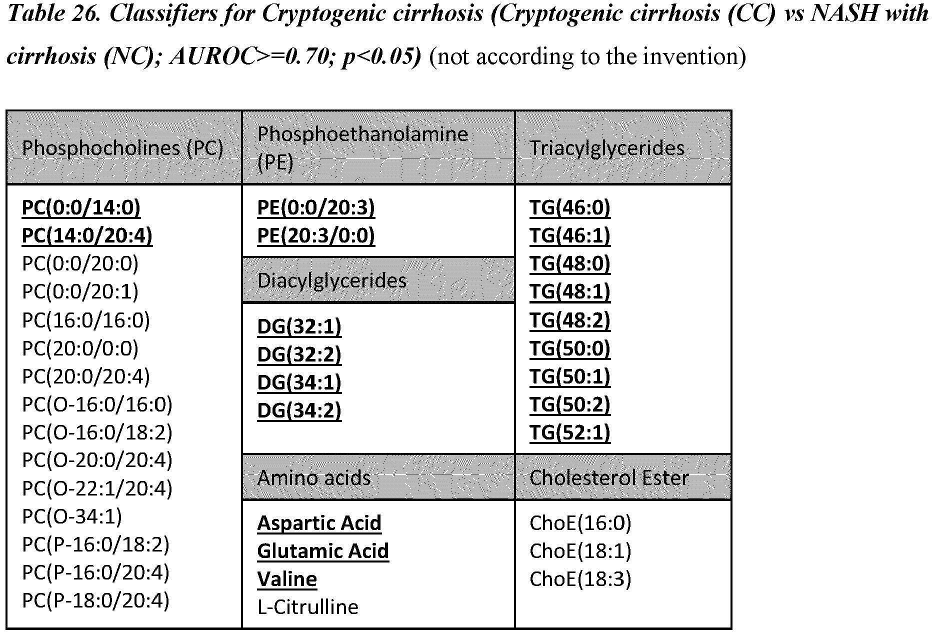

Underlined and bold: lower in CC; otherwise higher in CC

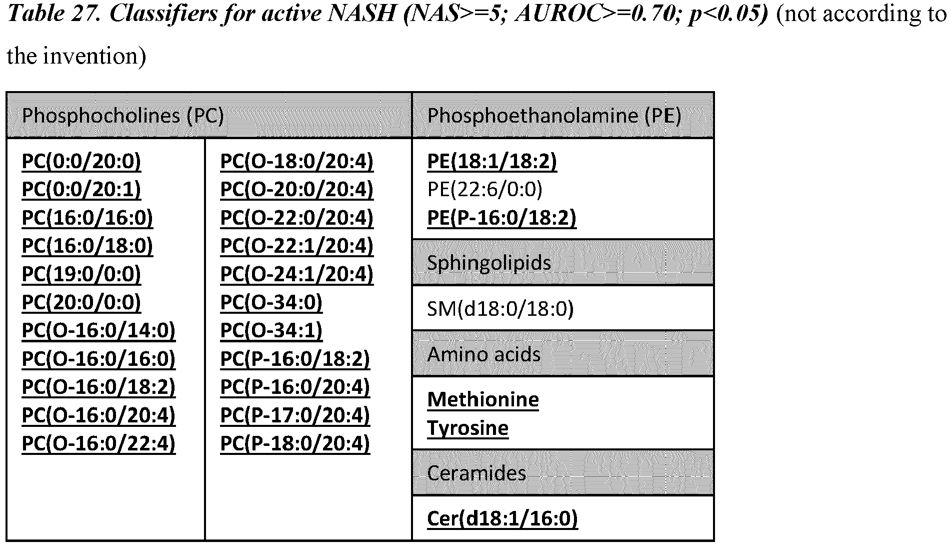

Underlined and bold: lower in NAS>=5; otherwise higher in NAS>=5

Unless otherwise defined, all technical and scientific terms used herein have the same meaning as commonly understood by one of ordinary skill in the art to which this invention belongs.

## Claims

1. A method for classifying a non-alcoholic steatohepatitis (NASH) patient with severe fibrosis, comprising:
measuring the protein levels of Collectin Kidney 1 (CL-K1), Spondin-1(RSPO1), Matrix metallopeptidase 7 (MMP-7), Complement component 7 (C7), Insulin-like growth factor binding protein 7 (IGFBP7), Interleukin 5 receptor subunit alpha (IL-5Ra), and Thrombospondin-2 (TSP2) in a serum sample isolated from the NASH patient;
determining whether Nonalcoholic Steatohepatitis Clinical Research Network (CRN) fibrosis stage of liver fibrosis in the NASH patient is stage 3 or 4, based on the measured protein levels and predetermined reference values, wherein the predetermined reference values include reference protein levels for each of Collectin Kidney 1 (CL-K1), Spondin-1(RSPO1), Matrix metallopeptidase 7 (MMP-7), Complement component 7 (C7), Insulin-like growth factor binding protein 7 (IGFBP7), Interleukin 5 receptor subunit alpha (IL-5Ra), and Thrombospondin-2 (TSP2) at CRN fibrosis stages 0 to 4; and
classifying the NASH patient with severe fibrosis if the determined CRN fibrosis stage is 3 or 4.

## Patentansprüche

1. Verfahren zur Klassifizierung eines Patienten mit nicht-alkoholischer Steatohepatitis (NASH) mit schwerer Fibrose, umfassend:
Messen der Proteinspiegel von Collectin Kidney 1 (CL-K1), Spondin-1 (RSPO1), Matrix-Metallopeptidase 7 (MMP-7), Komplementkomponente 7 (C7), Insulin-ähnliches Wachstumsfaktor-Bindungsprotein 7 (IGFBP7), Interleukin-5-Rezeptor-Untereinheit alpha (IL-5Ra) und Thrombospondin-2 (TSP2) in einer von dem NASH-Patienten isolierten Serumprobe;
Bestimmen, ob das Fibrosestadium der Leberfibrose des NASH-Patienten gemäß dem Clinical Research Network (CRN) über die nicht-alkoholischen Steatohepatitis das Stadium 3 oder 4 ist, basierend auf den gemessenen Proteinspiegeln und vorbestimmten Referenzwerten, wobei die vorbestimmten Referenzwerte Referenzproteinspiegel für jedes von Collectin Kidney 1 (CL-K1), Spondin-1 (RSPO1), Matrix-Metallopeptidase 7 (MMP-7), Komplementkomponente 7 (C7), Insulin-ähnliches Wachstumsfaktor-Bindungsprotein 7 (IGFBP7), Interleukin-5-Rezeptor-Untereinheit alpha (IL-5Ra) und Thrombospondin-2 (TSP2) in den CRN-Fibrosestadien 0 bis 4 einschließen; und
Klassifizierung des NASH-Patienten mit schwerer Fibrose, wenn das ermittelte CRN-Fibrosestadium 3 oder 4 ist.

## Revendications

1. Procédé de permettant de classifier un patient atteint de stéatohépatite non alcoolique (NASH) comme souffrant d'une fibrose sévère, comprenant les étapes consistant à :
mesurer les niveaux de protéine de collectine 1 du rein (CL-K1), de spondine-1 (RSPO1), de la métallopeptidase matricielle 7 (MMP-7), du composant 7 du complément (C7), de la protéine 7 liant un facteur de croissance analogue à l'insuline (IGFBP7), de la sous-unité alpha du récepteur de l'interleukine-5 (IL-5Ra), et de la thrombospondine-2 (TSP2) dans un échantillon de sérum isolé du patient atteint de NASH,
déterminer si le stade de fibrose de la fibrose hépatique chez le patient atteint de NASH est de stade 3 ou 4 selon le réseau de recherche clinique (CRN) sur la stéatohépatite non alcoolique, sur la base des niveaux de protéine mesurés et de valeurs de référence prédéterminées, dans lequel les valeurs de référence prédéterminées incluent des niveaux de protéine de référence pour chacun de la collectine 1 du rein (CL-K1), de la spondine-1 (RSPO1), de la métallopeptidase matricielle 7 (MMP-7), du composant 7 du complément (C7), de la protéine 7 liant un facteur de croissance analogue à l'insuline (IGFBP7), de la sous-unité alpha du récepteur de l'interleukine-5 (IL-5Ra), et de la thrombospondine-2 (TSP2) à des stades de fibrose CRN de 0 à 4, et
classifier le patient atteint de NASH comme présentant une fibrose sévère si le stade de fibrose CRN déterminé est de 3 ou 4.
